# EUROPEAN PATENT APPLICATION

(11) **EP 2 463 653 A1**
(43) Date of publication of application: **13.06.2012**
(21) Application number: 10806190.4
(22) Date of filing: 15.07.2010
(51) Int. Cl.: G01N 33/48, C12N 5/00, C12N 5/10, G06T 1/00

(54) **TECHNIQUE FOR CLASSIFYING CELLS, IMAGE PROCESSING PROGRAM AND IMAGE PROCESSING DEVICE USING THE TECHNIQUE, AND METHOD FOR PRODUCING CELL MASS**

(30) Priority: 07.08.2009 JP 2009184875
(71) Applicant: Nikon Corporation, Tokyo 100-8331 (JP)
(72) Inventor: ITO, Kei, Tokyo 100-8331 (JP); MIMURA, Masafumi, Tokyo 100-8331 (JP); YANO, Kazuhiro, Tokyo 100-8331 (JP); SASAKI, Hideki, Tokyo 100-8331 (JP)
(74) Representative: HOFFMANN EITLE
(86) International application number: PCT/JP2010/004600
(87) International publication number: WO 2011/016189

(57) **Abstract**

An image processing program AP comprises: a step (S1) of reading time-lapse images; a step (S2) of extracting cells in a viewing image from each time point and performing labeling and cell association; and a step (S3) of determining the maturity of a cell included in the viewing image from a designated time point t_{c}. In step (S4), an ID is assigned for a cell determined to be mature, and in step (S5), ID inheritance is performed by cell tracking of the time-lapse images in the backward or forward direction of time along the time axis. In step (S6), the number of unifications of cells is computed, and cells are classified on the basis of the inherited ID information, and the results of analysis are outputted in step (S7).

## Description

### TECHNICAL FIELD

The present invention relates to a cell classification method for classifying cells from a time-lapse image taken in cell viewing.

### TECHNICAL BACKGROUND

Living plant and animal cells are used to evaluate cell culture environments or the efficacy of drugs, and numerous cells are required for a test sample. Cell culturing is therefore performed to culture and cause proliferation of living cells. In cell culturing, since some cells in the cell culture die, and in the case of ES cells or iPS cells, sociality is not maintained and proliferation does not occur unless culturing is begun with a certain aggregation of a plurality of cells, a single medium is typically seeded with multiple cells for cell culturing. A cell culture microscopy is a typical example of a device for viewing the progress of a cell culture.

A cell culture microscopy is provided with a cell culture device for creating an environment suitable for cell culturing, and a micro viewing system for microscope viewing of a cell in a cell culture container, and is configured so that that status of cell division, unification, differentiation, and the like can be viewed while a living cell is being cultured (refer to Patent Document 1, for example). Cells (cell aggregations) cultivated by cell culturing are sorted by extracting colonies by sensory evaluation according to the appearance of cells viewed through a microscope at regular time intervals by an observer.

### PRIOR ARTS LIST

### PATENT DCCUMENTS

Patent Document 1: Japanese Laid-open Patent Publication No. 2004-229619(A)

### SUMMARY OF THE INVENTION

### PROBLEMS TO BE SOLVED BY THE INVENTION

In order to increase the precision of evaluation in evaluation of cell culture environment or drug efficacy, there is a need for characteristics of test samples to be aligned (uniform), and ideally, cultured cells that originated from a single cell are preferably used. However, in conventional cell culturing, mature cells are extracted by sensory evaluation according to cell appearance when viewed by an observer through a microscope, and the origin (e.g., whether the cell formed by unification of several cells) of a matured cell is not the subject of evaluation. It is also difficult to view cells over a long continuous period with adequate frequency, and colonies often merge with one another during observation gaps in which there is no precognition by the observer. An accurate evaluation is therefore difficult to obtain in cases in which there are differences in drug effect and other characteristics between samples despite having a plurality of mature cell samples extracted.

The present invention was developed in view of such problems as the foregoing, and an object of the present invention is to provide a means whereby cells can be sorted and evaluated according to the configuration of the cells.

### MEANS TO SOLVE THE PROBLEMS

A first aspect of the present invention is a cell classification method. This cell classification method comprises the steps of: extracting cells (meaning cells in integrated form, including cell aggregations in which a plurality of cells is unified and integrated) included in the image from a first image taken at a predetermined time point; extracting cells included in the image from a second image taken a predetermine time apart from the predetermined time point; associating the cells extracted from the first image and the cells extracted from the second image, assigning pre-integration cell information to an integrated cell in the case that a plurality of cells of the first image is unified in the second image, and assigning pre-separation cell information to each separated cell in the case that a single cell in the first image is separated into a plurality of cells in the second image; executing the extraction and association of cells while sequentially shifting the first image and the second image along a time axis for time-lapse images, and causing the cell information of the cells included in the images to be sequentially inherited; and classifying cells on the basis of the inherited cell information for the cells included in an image taken at an arbitrary time point.

A second aspect of the present invention is an image processing program for causing a computer to function as an image processing device for obtaining an image in which cells are photographed by an imaging device and performing image professing, the image processing program being readable by the computer. The image processing program comprises: a first step of obtaining a first image taken at a predetermined time point by the imaging device and extracting cells included in the image; a second step of obtaining a second image taken a predetermined time apart from the predetermined time point by the imaging device and extracting cells included in the image; a third step of associating the cells extracted from the first image and the cells extracted from the second image, assigning pre-integration cell information to the integrated cell in the case that a plurality of cells of the first image is integrated into a single cell in the second image, and assigning pre-separation cell information to the separated cells in the case that a single cell of the first image is separated into a plurality of cells in the second image; a step of executing the first through third steps for time-lapse images while sequentially shifting the first through third steps along the time axis, and causing the cell information of the cells included in each image to be sequentially inherited; and a step of outputting the inherited cell information for cells included in an image taken at a designated time point. This image processing program preferably further comprises a step of classifying, on the basis of the inherited cell information, the cells included on the image taken at the designated time point; and a step of outputting a classification result.

A third aspect of the present invention is an image processing device comprising: an image analyzer for obtaining time-lapse images in which cells are photographed at a predetermined time interval by an imaging device; and an output unit for outputting results of analysis by the image analyzer. In the image processing device, the image analyzer extracts cells included in the image from a first image taken at a predetermined time point; extracts cells included in the image from a second image taken a predetermined time apart from the predetermined time point; associates the cells extracted from the first image and the cells extracted from the second image, assigns pre-integration cell information to an integrated cell in the case that a plurality of cells of the first image is unified in the second image, assigns pre-separation cell information to each separated cell in the case that a single cell in the first image is separated into a plurality of cells in the second image; executes the extraction and association of cells while sequentially shifting the first image and the second image along a time axis for time-lapse images, and causes the cell information of the cells included in the images to be sequentially inherited; and the output unit outputs the inherited cell information for the cells included in an image taken at a designated time point. In the image processing device, a configuration is preferably adopted in which the image analyzer classifies the cells included in the image taken at the designated time point on the basis of the inherited cell information, and the output unlit outputs the results of classification by the image analyzer.

In the present invention according to the first through third aspects described above, preferably, inheritance of the cell information is executed in the backward direction of time to the start time of viewing, along the time axis of the time-lapse images, the predetermined time point at which the first image is taken being a time point t, and the time point at which the second image is taken being a time point t-1 the predetermined time prior to the predetermined time point; and the cells in the image taken at the arbitrary time point are classified according to the number of cells that are origin cells constituting each cell, on the basis of the cell information of each cell inherited back to the start time of viewing.

Also preferably, inheritance of the cell information is executed in the forward direction of time from the start time of viewing, along the time axis of the time-lapse images, the predetermined time point at which the first image is taken being a time point t, and the time point at which the second image is taken being a time point t+1 the predetermined time after the predetermined time point; and the cells included in a viewing image taken at the arbitrary time point are classified according to the number of cells that are origin cells constituting each cell, on the basis of the cell information of each cell inherited up to the time point.

### ADVANTAGEOUS EFFECTS OF THE INVENTION

In the cell classification method, image processing program, and image processing device of the present invention, cells are extracted and associated while first and second images are sequentially shifted along the time axis, cell unification or division is sequentially inherited as cell information of each cell, and cells are classified on the basis of the inherited cell information. Consequently, through the present intention, the configuration of cultured cells is clearly evident, and a means can be provided whereby cells can be sorted and evaluated.

By a method in which inheritance of cell information for time-lapse images is executed in the backward direction of time along the time axis to the start time of viewing, the number of cells at the start of tracking can be reduced. The processing burden for calculation can therefore be reduced, and processing can be carried out at high speed. By a method in which inheritance of cell information is executed in the forward direction of time along the time axis of the time-lapse images from the start time of viewing, such characteristics as the number of origin cells of each cell at the current time can be monitored in real time.

### BRIEF DESCRIPTION OF THE DRAWINGS

FIG. 1 is a flowchart showing an example of the image processing program AP1 for automatically selecting mature cells and performing image analysis;
FIG. 2 is a rough structural view showing the cell culture viewing system as an example of an application of the present invention;
FIG. 3 is a block diagram showing the cell culture viewing system;
FIG. 4 is a block diagram showing an example of the overall configuration of the image processing device;
FIG. 5 is a flowchart showing the image processing program GP1 for analyzing time-lapse images in the backward direction of the time axis;
FIG. 6 is a schematic view showing the image analysis performed by the image processing program GP1 for time-lapse images taken by the imaging device;
FIG. 7 is a flowchart of a case in which ID inheritance by tracking is applied to the image processing program GP2 for analyzing time-lapse images in the forward direction of time;
FIG. 8 is a schematic view showing the image analysis performed by the image processing program GP2 for time-lapse images taken by the imaging device;
FIG. 9 is a flowchart showing the image processing program SP1 for detecting stratified portions as part of the image processing programs GP1, GP2;
FIG. 10A shows the first image and FIG. 10B shows the second image of a cell aggregation photographed at a predetermined time interval;
FIG. 11A shows an example of the configuration of the local region set in the first image, and FIG. 11B is a view showing the execution of block matching for a neighborhood that includes the corresponding position in the second image;
FIG. 12A is a view showing an example of the size of the local regions with respect to the cell aggregation, and FIG. 12B shows the distribution of stratification degrees computed by image processing in a manner that is visually recognizable by black-to-white gradations;
FIG. 13 is a flowchart showing the image processing program SP2 for determining the maturity of a cell aggregation on the basis of a time-lapse variation of a summation of stratification degrees;
FIG. 14 is a graph of the temporal variation of the summation of stratification degrees;
FIG. 15 is a flowchart showing the image processing program SP3 for determining the maturity of a cell aggregation on the basis of the time-lapse variation of the occupancy ratio of stratified portions;
FIG. 16 is a graph of the temporal variation of the occupancy ratio of stratified portions;
FIGS. 17A-17B are schematic views showing viewing images of a cell aggregation, where FIG. 17A shows the initial stage of cell culturing and FIG. 17B shows a mature state in which the stratified region has spread to the entire area;
FIG. 18 is a flowchart showing the image processing program SP4 for determining the maturity of a cell aggregation on the basis of the time-lapse variation of the luminance summation near the contour of the cell aggregation;
FIG. 19 is a graph of the temporal variation of the luminance summation near the contour of the cell aggregation;
FIG. 20 is a flowchart showing the image processing program SP5 for determining the maturity of a cell aggregation on the basis of the time-lapse variation of the complexity of the contour shape of the cell aggregation;
FIG. 21 is a graph of the temporal variation of the complexity of the contour of the cell aggregation;
FIG. 22 shows an example of the user interface for displaying the results of analysis; and
FIG. 23 is a flowchart showing the method for producing a cell aggregation.

### DESCRIPTION OF THE EMBODIMENTS

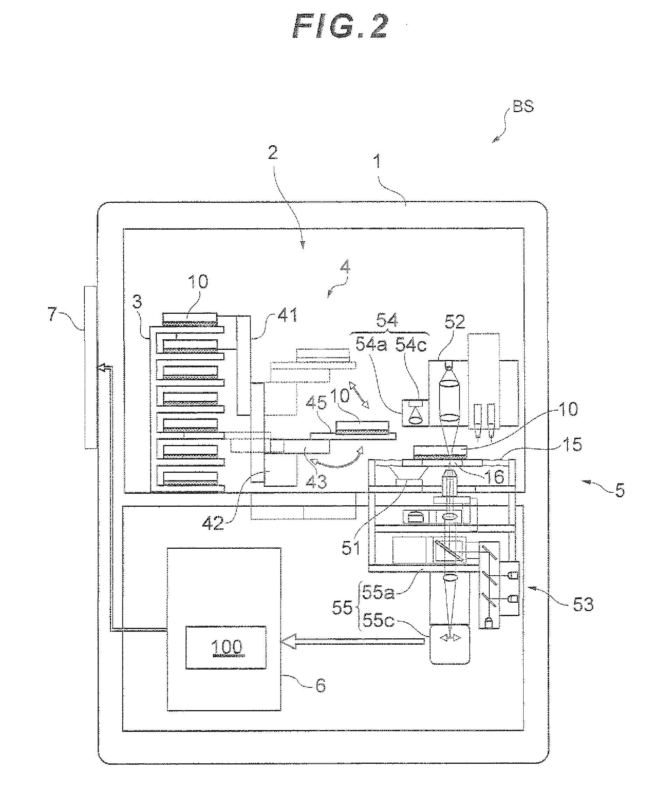

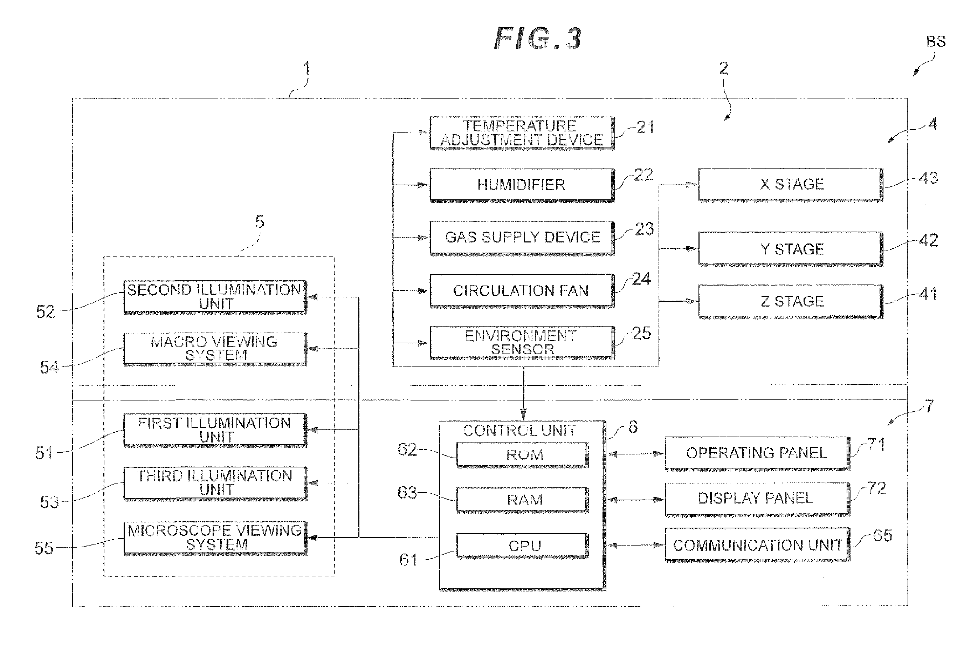

Embodiments of the present invention are described below with preference to the accompanying drawings. FIGS. 2 and 3 are a rough structural view and a block diagram, respectively, showing a cell culture viewing system as an example of a system to which the image processing device of the present invention is applied. The overall configuration of the cell culture viewing system BS will first be briefly described.

The cell culture viewing system BS is primarily composed of a cell culture chamber 2 provided at the top of a chassis 1; a stocker 3 for accommodating and retaining a plurality of cell culture containers 10; a viewing unit 5 for viewing samples in the cell culture containers 10; a conveyance unlit 4 for conveying the cell culture containers 10; a control unit 6 for controlling the operation of the system; an operating board 7 provided with an image display device; and other components.

The cell culture chamber 2 is a room for forming a cell culture environment, and the cell culture chamber 2 is additionally provided with such components as a temperature adjustment device 21, a humidifier 22 a gas supply device 23 for supplying CO₂ gas, N₂ gas, or other gas, a circulation fan 24, and an environment sensor 25 for detecting the temperature, humidity, and other characteristics of the cell culture chamber 2. The stocker 3 is formed in a shelf shape having a plurality of divisions in the front-rear and up-down direction, and a specific number is set for each shelf. An appropriate cell culture container 10 is selected according to the type or purpose of the cell to be cultured, and cell samples are injected together with a culture medium and retained in dish-type cell culture containers, for example. A code number is assigned to each cell culture container 10, and each cell culture container 10 is associated with a designated number and accommodated in the stocker 3. The conveyance unit 4 is composed of such components as a Z stage 41 provided within the cell culture chamber 2 so as to be able to move up and down, a Y stage 42 attached so as to be able to move forward and backward, and an X stage 43 attached so as to be able to move left and right, and a support arm 45 for lifting and supporting a cell culture container 10 is provided toward the distal end of the X stage 43.

The viewing unit 5 is composed of such components as a first illumination unit 51 for illuminating a sample from below a sample stage 15; a second illumination unit 52 for illuminating a sample along the optical axis of a micro viewing system 55 from above the sample stage 15; a third illumination unit 53 for illuminating the sample from below the sample stage 15; a macro viewing system 54 for macro viewing of the sample; a micro viewing system 55 for micro viewing of the sample; and an image processing device 100. A transparent window 16 is provided to the sample stage 15 in the region of viewing by the micro viewing system 55.

The macro viewing system 54 has a viewing optical system 54a and a CCD camera or other imaging device 54c for capturing an image of a sample that is imaged by the viewing optical system, and the macro viewing system 54 obtains an overall viewing image (macro image) from above the cell culture container 10 which is backlight-illuminated by the first illumination unit 51. The micro viewing system 55 has a viewing optical system 55a composed of an objective, an intermediate zooming lens, a fluorescence filter, and other components; and a cooled CCD camera or other imaging device 55c for taking an image of the sample imaged by the viewing optical system 55a. A plurality of objectives and intermediate zooming lenses is provided, and configured so that an arbitrary viewing magnification can be set by varying the combination of lenses. The micro viewing system 55 obtains a transmission image of a cell illuminated by the second illumination unit 52, a reflection image of a cell illuminated by the third illumination unit 53, a fluorescence image of a cell illuminated by the third illumination unit 53, or another microscope viewing image (micro image) of a microscopically viewed cell in the cell culture container 10.

The image processing device 100 processes signals taken by the imaging device 54c of the macro viewing system 54 and the imaging device 55c of the micro viewing system 55 and inputted from these imaging devices, and generates an overall viewing image, micro viewing image, or other image. The image processing device 100 applies image analysis to the viewing images (image data), and generates a time lapse image, analyzes the activity state of a cell, analyzes the maturity of a cell, analyzes the configuration of a cell, and performs other processing. The image processing device 100 will be described in detail hereinafter.

The control unit 6 has a CPU 61 for executing processing, a ROM 62 including a hard disk, DVD, or other auxiliary storage device, in which a control program, control data, and the like are set and stored for the cell culture viewing system BS; a RAM 63 for temporarily storing viewing conditions, image data and the like; and other components, and the control unit 6 controls the operation of the cell culture viewing system BS. The components including the cell culture chamber 2, conveyance unlit 4, viewing unit 5, and operating board 7 are therefore connected to the control unit 6, as shown in FIG. 3. In accordance with a viewing program, the RAM 63 stores environment conditions of the cell culture chamber 2, a viewing schedule, and viewing classifications, viewing positions, viewing magnifications, and other information for the viewing unit 5. The RAM 63 is also provided with an image data storage region for recording image data captured by the viewing unit 5, and index data which include a code number of the cell culture container 10, an image capture time, and other information are recorded in association with image data.

The operating board 7 is provided with an operating panel 71 to which a keyboard, switch, or other input/output instrument is provided, and a display panel 72 for displaying an operating screen, a viewing images, analysis results, and the like, and settings or conditions of the viewing program are selected, and operating commands and the like are inputted in the operating panel 71. A communication unit 65 is configured according to a wired or wireless communication standard, and control signals and viewing data can be transmitted to and received from a computer or the like that is externally connected to the communication unit 65.

In the cell culture viewing system BS thus generally configured, the CPU 61 controls the operation of each component and automatically photographs the sample in the cell culture container 10, in accordance with the viewing program set in the operating board 7. When the viewing program is started, the CPU 61 controls the operation of the temperature adjustment device 21, humidifier 22, and other components to control the environment of the cell culture chamber 2, on the basis of the environment conditions stored in the RAM 63. The CPU 61 also reads a viewing condition stored in the RAM 63, operates the X, Y, and Z stages 43, 42, 41 on the basis of the viewing schedule, conveys the cell culture container 10 to be viewed from the stocker 3 to the sample stage 15, and initiates viewing by the viewing unit 5. For example, in a case in which the viewing set in the viewing program is micro viewing of a cell, the cell culture container 10 is positioned on the optical axis of the micro viewing system 55, the light source of the second illumination unit 52 or the third illumination unit 53 is lit, and a micro viewing image is captured by the imaging device 55c.

In the cell culture viewing system BS configured as described above, the image processing device 100 has the function of taking images of a cultured cell at predetermined time intervals by the imaging devices (54c, 55c), analyzing the taken images, and outputting information (cell configuration information or classification information) that is useful for sorting and evaluation of cultured cells. This function is suitable for use in research with iPS cells, ES cells, and other cells.

This function is performed using tracking of a cell in time-lapse images, and is realized by assigning information (cell information) of a cell prior to unification or dividing thereof to a cell that has unified or divided when a unification or division of a cell has occurred between adjacent images, thus creating an inheritance of cell information by shifting the sequence of cell information in the forward time direction or the backward time direction of the time axis of the time-lapse images, and outputting a display of the inherited cell information for a cell in an image from an arbitrary time point or displaying a classification result that is based on the inherited cell information.

For example, in a case in which cells c1, c2 that are two cells in a viewing image (first image) from time point t are unified in a viewing image (second image) from the next time point t+1, pre-integration cell information c1, c2 is assigned to the integrated cell, and by cremating an inheritance of cell information by shifting the sequence of cell information in the forward time direction of the time axis of the time-lapse images, for a cell in the viewing image from time point t+x, for example, an output is displayed showing that the cell is composed of three cells c1, c2, c5 from time point t.

Here, by setting the time point t as t=0, which is the start time of viewing, it is possible to determine which of several origin cells the cells in the viewing image from time t+x are composed of, and whether a colony formed from a single cell is present. By continuing viewing with time point t+x as the present time (most recant viewing time), the facts described above can be known in real time, and cells can be precisely evaluated and sorted by understanding the origin and configuration of growing cell aggregations.

In a case in which a cell C1 that is a single cell in the viewing image (first image) from time point t is two cells in the viewing image (second image) from the previous time point t-1, pre-separation cell information C1₁, C1₂ is assigned to each of the two separated cells, and by creating an inheritance of cell information by shifting the sequence of cell information in the backward time direction of the time axis of the time-lapse images, an output is displayed showing that cells C1₁, C1₂, C1₃ which are one cell C1 at time point t are present in the viewing image from time point t-x, for example.

At this time, by setting the time point t-x as t=0, which is the start time of viewing, it is possible to determine which of several cells present at the start of viewing that the cells of a cell aggregation growing at time point t are composed of, and whether cells are present which form a colony from a single cell, and by setting time point t as the present time (most recent viewing time), cells can be precisely evaluated and sorted by assessing the origin and configuration of cell aggregations that are being cultured.

For cell aggregations that appear in a viewing image from a specified time point within a viewing period, examples of cell classification based on cell information such as described above include displaying cell aggregations in the viewing image in different colors according to the number of cells in each cell aggregation, displaying cell aggregations that include specific origin cells in different colors, and other classifications. Other examples include adding determination of maturity of cell aggregations as described hereinafter and providing the color-coded display described above only for cell aggregations that are determined to be mature, or rather than providing a color-coded display, displaying a frame around cell aggregations that are determined to be mature. An output display of a histogram in which the cell aggregations in a viewing image are classified by number of constituent cells is also effective for obtaining an overview of the cultured cells.

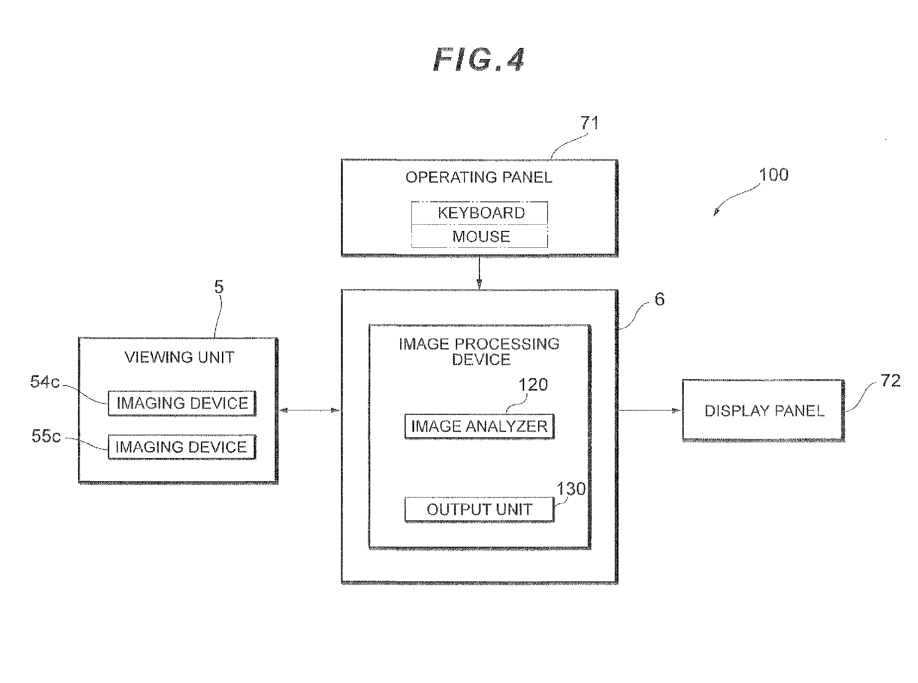

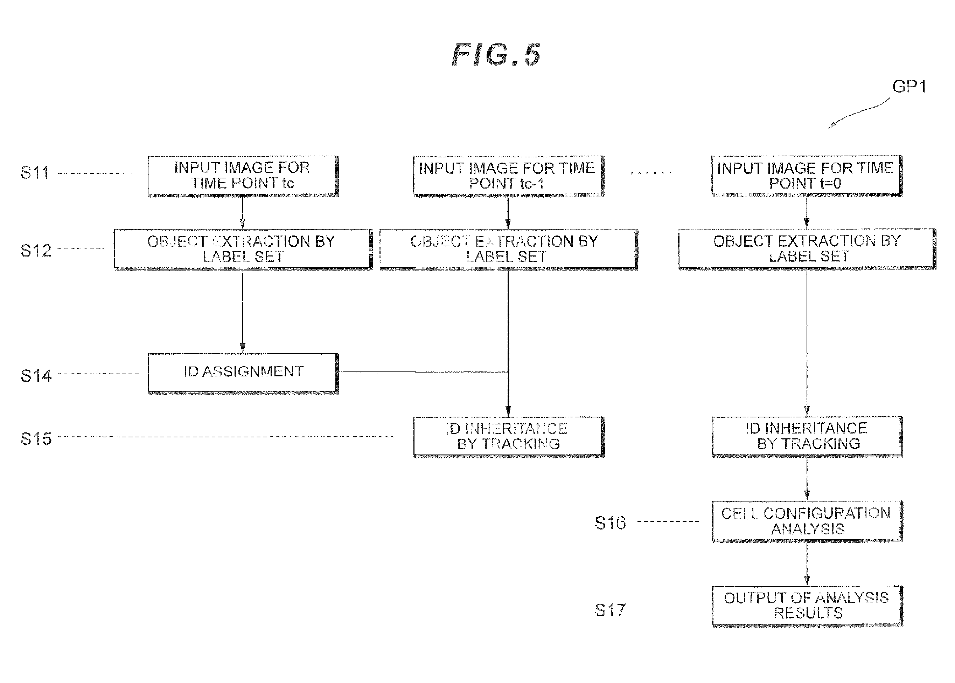

Time-lapse image analysis using the method described above is executed in the image processing device 100. FIG. 4 is a rough block diagram of the image processing device 100, and FIG. 5 is a flowchart showing the image processing program GP1 used in the case of analyzing the time-lapse images in the backward direction of the time axis, in the image processing program GP executed in the image processing device 100.

In the image processing device 100, an image processing program GP (GP1, GP2) set and stored in the ROM 62 is read by the CPU 61, and processing based on the image processing program GP is executed in sequence by the CPU 61. In other words, the image processing program GP is software for causing the CPU 61 (computer), which is a hardware resource, to function as the image processing device 100.

An image analyzer 120 applies image processing to time-lapse viewing images taken by an imaging device (in this description, the imaging device 55c of the micro system) and recorded in the RAM 63.

### (I) Method for Computing the Number of Origin Cells by Tracking Backward Along the Time Axis

When the image processing program GP1 is started by an operational input to the operating panel 71 or by other means, in the first step S11, the image analyzer 120 reads and obtains a first image taken at a time point t=t_{c} (e.g., the latest observation timing) specified by the observer, and a second image taken at a prior time point t=t_{c}-1 from time-lapse images stored in the RAM 63, and in step S12 extracts cells included in each image. A Snakes, Level Set, or other dynamic contouring or a dispersion filter is used to extract the cells from the viewing images (image data) .

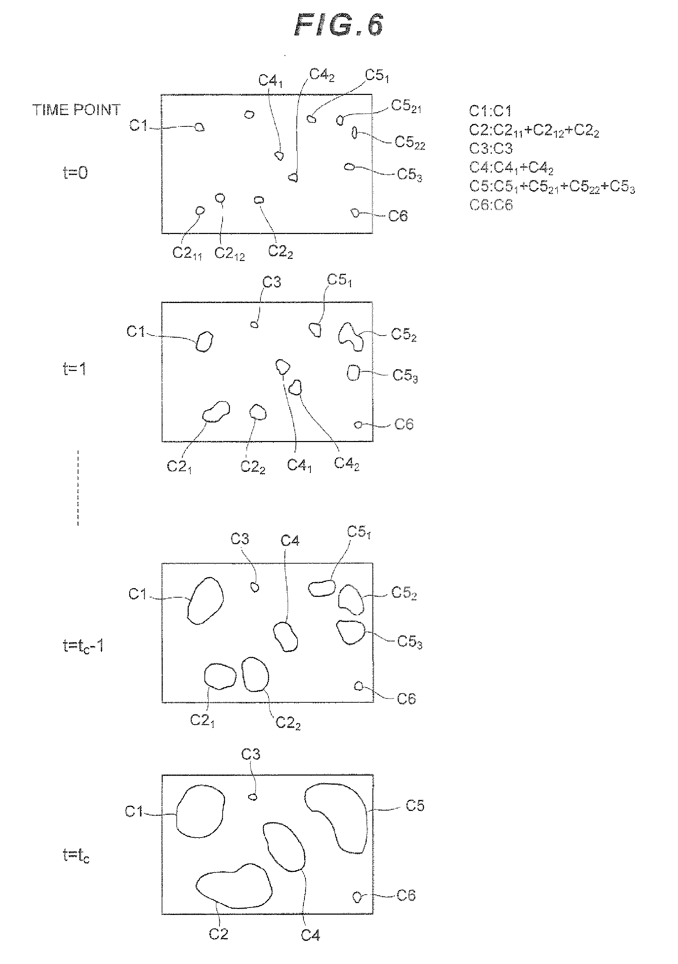

In the next step S14, the cells extracted in the first image are each assigned a label (ID). For example, in the time-lapse images shown schematically in FIG. 6 from time points t=0, 1, ... , t_{c}-1, t_{c} stored in the RAM 63, the cells in the first image from time point t=t_{c} are assigned the IDs (identification numbers) C1, C2, C3, and so on to C6.

Then, in step S15, cells in the first image and second image that correspond to each other are determined, and inheritance of cell information by tracking is performed. At this time, pre-integration cell information is inherited by the integrated cells in a case in which a plurality of cells of the first image is unified in the second image, and in a case in which a single cell in the first image is separated into a plurality of cells in the second image, the pre-separation cell information is inherited by the separated cells.

Inheritance of cell information is determined by overlap between the region in which a cell is present in the first image and the region in which the cell is present in the second image, and the cell information of the first image is inherited by cells which overlap even partially. The capture interval for viewing images is generally set adequately small with respect to the movement speed of cells, and tracking using optical flow, or Kalman filtering or another linear prediction method is used for tracking. In such cases as when the viewing interval is inadequate and there is no overlap of cell regions between the first image and the second image, tracking can be performed by correlation values of distances to cells in the previous frame, or by using Kalman filtering or other linear prediction, or extended Kalman filtering, particle filtering, or other non-linear movement prediction.

In a case in which the cell C2 in the first image from time point t=t_{c} is separated into two cells in the second image from time point t=t_{c}-1 (two cells are unified over time) as shown in FIG. 6, the ID of the cell C2 is inherited as the cell information, and IDs such as C2₁ and C2₂ are assigned to the cells. The cell C5 in the first image is divided in the same manner into three in the second image, in which case the IDs C5₁, C5₂, and C5₃ inherited form the ID of the cell C5 are assigned as cell information to the cells of the second image.

The steps S11 through S15 described above are subsequently executed for the time-lapse images while the first image and second image are sequentially shifted in the backward direction of the time axis, and the cell information of each cell in the images is sequentially inherited until the time point t=0. Specifically, in the next cell information inheritance processing, cells are tracked with the viewing image obtained at time point t=t_{c}-1 as the first image and the viewing image obtained at time point t=t_{c}-2 as the second image, and cell information is inherited by the cells of time point t_{c}-2 whose regions overlap even partially with the cells of time point t_{c}-1 which have already inherited cell information.

This inheritance of cell information is repeated until the second image becomes the viewing image of time point t=0, and the cell information for time point t=0 is derived. In step S16, the number of cells that have the same cell information as at the cell seeding time t=0 are counted based on the inherited cell information; classification, computation of the number of constituent cells and the origin cells of cells that are integrated at time point t_{c}, and other operations are performed, and the results of analysis are outputted from an output unlit 130 to the display panel 72 or elsewhere in step S17.

FIG. 6 shows an output example in which the inherited cell information is displayed as assigned to the cells of the viewing image for each time point, and shows an output example (classification chart at the top right of the drawing) in which the inherited cell information is organized, and the origin cells that constitute the cells C1 through C6 at time point t_{c} are classified and displayed.

In the former output example, in which the cell information is displayed as assigned to the cells in the viewing images, the cell composition can be made more easily understandable by displaying the cells C5₁, C5₂₁, C5₂₂, C5₃ that constitute the cell C5 at time point t_{c} in the same color, and displaying the cells C4₁, C4₂ that constitute the cell C4 at time point t_{c} in a different color, for example, or otherwise classifying the display for each cell group that constitutes the cells C1 through C6 at time point t_{c}. In the latter output example, the classification of the origin cells of the cells C1 through C6 may be displayed together with the viewing image from time point t_{c} so as to be beside or elsewhere in relation to the viewing image, or the cells C1 through C6 may be color coded according to the number (1 to n) of origin cells. Displaying the output in this manner enables easier comprehension of the cell configuration.

Displaying the cell information and displaying a classification of the origin cells on the basis of the cell information in this manner enables an observer to accurately determine that the cell C1 forming a mature cell aggregation at time point t_{c} is a colony formed from a single cell, or that the cells C2, C4, C5 which appear as cell aggregations that matured in the same manner in the viewing image from time point t_{c} are composed of three, two, and four origin cells each.

A configuration is described above in which tracking is performed for all the cells included in the viewing image from time point t_{c}, and cell information or cell classification is outputted for all the cells, but a configuration may also be adopted in which processing is executed for an analysis subject selected from the viewing image from time point t_{c}. For example, a partial region for which to perform analysis may be designated from the viewing image as a whole using a mouse or the like, a grown cell (e.g., cell C1, C2, C4, or C5 in FIG. 6) may be selected from the viewing image using a mouse or the like, or a mature cell may be automatically selected for analysis by using the cell maturation determination method described in detail hereinafter. Immature cells which do not need to be analyzed can thereby be excluded to reduce the processing burden, and origin information for desired cells can be analyzed at high speed.

### (II) Method for Computing the Number of Origan Cells by Tracking Along the Time Axis

The method for computing the number of origin cells by tracking in the forward direction of the time axis from the start of viewing will next be described. The image processing program GP2 of this method has the same basic configuration as the flowchart for the image processing program GP1 shown in FIG. 5, and is realized by tracking in the forward direction of the time axis by replacing "input image for time point t_{c}" in FIG. 5 with "input image for time point t=0, " replacing "input image for time point t_{c}-1" with "input image for time point 1=1," and so on to replacing "input image for time point t=0" with "input image for time point t_{c}."

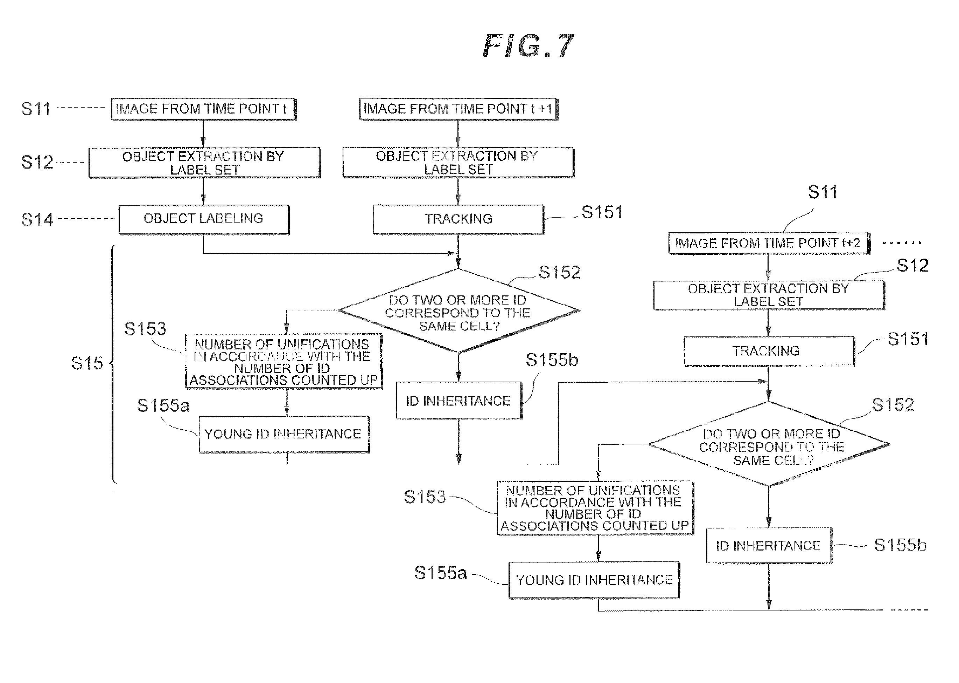

An example of a different configuration than the abovementioned image processing program GP1 for ID inheritance (inheritance of cell information) by tracking in step S15 of FIG. 5 is described below, and FIG. 7 is a detailed flowchart showing the application of the image processing program GP2 to this ID inheritance. In FIG. 7, the same step numbers are used for steps which perform the same processing as in the image processing program GP1.

When the image processing program GP2 is started by an operational input to the operating panel 71 or by other means, in the first step S11, a first image taken at time point t=0 (the viewing timing when an initial time point is specified by the observer) and a second image taken at the next time point t=1 are read and obtained from the time-lapse images stored in the RAM 63, and the cells included in each image are extracted in step S12.

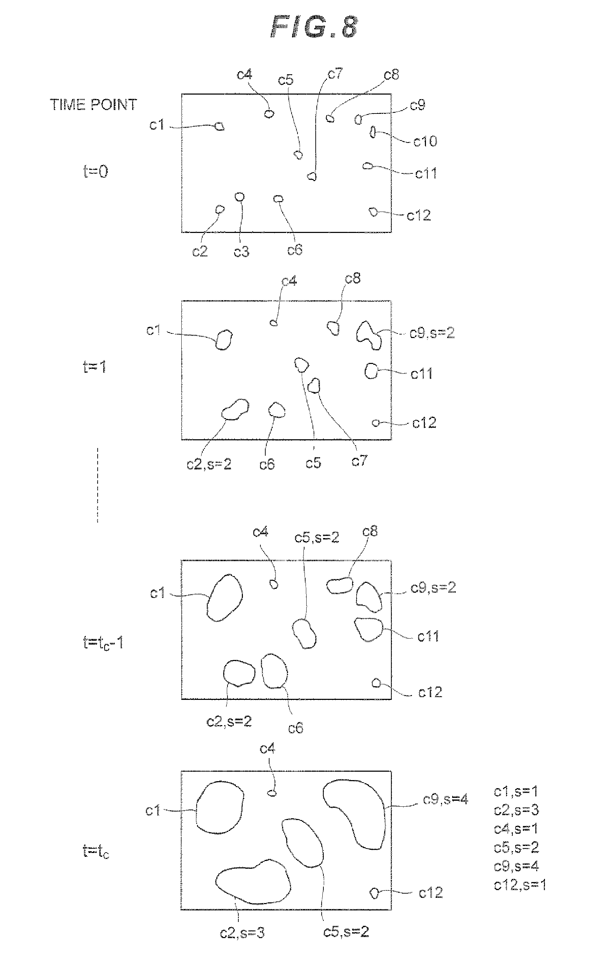

In the next step S14, the cells extracted in the first image are each assigned a label (ID). For example, in the time-lapse images shown schematically in FIG. 8 from time points t=0, 1, ..., t_{c}-1, t_{c} stored in the RAM 63, the cells in the first image from time point t=0 are assigned the IDs c1, c2, c3, and so on to c12.

Then, in step S15, cells in the first image and second image that correspond to each other are determined, and inheritance of cell information (ID) by tracking is performed. This ID inheritance by tracking is performed according to steps S151 through S155 in FIG. 7.

In step S151, tracking processing is performed, and cells for which a region occupied by a cell in the first image and a region occupied by a cell in the second image overlap even partially are associated as being the same cells. As previously mentioned, the capture interval for viewing images is set so as to be adequately small with respect to the movement speed of the cells, and cells can be associated by observing the overlap of cell regions. In such cases as when there is no overlap of cell regions between the first image and the second image, tracking can be performed by correlation values of distances to cells in the previous frame, or by using Kalman filtering or other linear prediction, or extended Kalman filtering, particle filtering, or other non-linear movement prediction.

In step S152, a determination is made as to whether two or more cells of the first image correspond to a single cell of the second image, and in the case that two or more cells of the first image are determined to correspond to a single cell of the second image (two or more cells have integrated), the process proceeds to step S153. Otherwise the process proceeds to step S155b. In step S153, the number of unifications in accordance with the number of associations of two or more IDs in the first image is counted up, one ID of either cell, e.g., the youngest (smallest) ID in the first image is assigned to the cell in the second image and the cell information is inherited in step S155a. In the case that a determination is made in step S152 that cells are not unified, i.e., when there is a one-to-one correspondence between the cells of the first image and the cells of the second image, or one cell in the first image is separated into two or more cells in the second, image, the IDs of the cells in the first image are inherited in step S155b without change.

For example, in a case in which cells c2 and c3 of the first image from time point t=0 are integrated into a single cell in the second image from time point t=1, as shown in FIG. 8, the younger number c2 of the two ID numbers and the number s = 2 of cell unifications are inherited as cell information by the integrated cell of the second image. Cells c9 and c10 of the first image are also integrated in the second image, and in this case, the ID number c9 and cell unification count s = 2 are inherited as cell information by the integrated cell in the second image.

The steps S11 through S15 described above are subsequently executed for the time-lapse images while the first image and second image are sequentially shifted in the forward direction of the time axis, and the cell information of each cell in the images is sequentially inherited until the designated time point (e.g., the nearest viewing timing for the present time point) t=t_{c}. Specifically, in the next cell information inheritance processing, cells are tracked with the viewing image obtained at time point t=1 as the first image and the viewing image obtained at time point t=2 as the second image, and cell information is inherited by the cells of time point t=2 whose regions overlap even partially with the cells of time point t=1 which have already inherited cell information.

This inheritance of cell information is repeated until the second image becomes the viewing image of time point t=t_{c}, and the cell information for time point t=t_{c} is derived. In step S16 (refer to FIG. 5), classification and computation of the number of constituent cells and the origin cells of cells in the viewing image from time point t_{c} are performed, and the results of analysis are outputted from the output unit to the display panel 72 or elsewhere in step S17.

Fig. 8 shows an output example in which the inherited cell information is displayed as assigned to the cells of the viewing image for each time point, and shows an output example (classification chart at the bottom right of the drawing) in which the inherited cell information is organized, and the numbers of origin cells that constitute the cells c1, c2, c4, c5, c9, c12 in the viewing image from time point t_{c} are classified and displayed. In the output example in which the cell information is displayed as assignee to the cells of the viewing image, the cells c1, c2, c4, c5, c9, c12 are shown color-coded according to the unification count s (s = 1 to n) of the inherited cell information, i.e., the number of origin cells that form each cell at time point t_{c}, and the configuration of each cell can thereby be more easily understood.

Displaying the cell information and displaying a classification of the origin cells on the basis of the cell information in this manner enables an observer to accurately determine that the cell c1 forming a mature cell aggregation at time point t_{c} is a colony formed from a single cell, or that the cells c2, c5, c9 which appear as cell aggregations that matured in the same manner in the viewing image from time point t_{c} are composed of three, two, and four origin cells each. By the method described above, using time point t_{c} as the nearest viewing timing, the number of origin or compositional cells of the cells viewed at the current time can be determined by continuous analysis that is updated each time a viewing image is taken, and cells in the cell culture can be precisely evaluated and sorted in real time.

A configuration is described above in which tracking is performed for all the cells included in the viewing image from time point t=0, and cell information or cell classification is outputted for all the cells, but a configuration may also be adopted in which processing is executed for an analysis subject selected from an initial viewing image from time point t=0, 1, 2, or another point. For example, a partial region for which to perform analysis may be designated from the viewing image as a whole using a mouse or the like, the cells c4, c12, and others that are at an initial stage and exhibit almost no growth may be excluded and other cells selected, or a mature cell may be automatically selected for analysis by using the cell maturation determination method described hereinafter. Immature cells which do not need to be analyzed can thereby be excluded to reduce the processing burden, and origin information for desired cells can be analyzed at high speed.

Following is a description of the cell maturation determination method used in the case that a cell that is adequately mature at a designated time point is automatically selected for analysis for the cell information display and classification display of origin cells described above. In the following description, the term "cell aggregation" will be used as appropriate to indicate a cell growth state.

In a plate culture in which cultured cells spread in a plane in the medium, multiple layers begin to form in the process in which iPS cells, ES cells, or other cells grow into a cell aggregation in which the cells have sociality, and the stratification spreads throughout the cell aggregation over the course of maturation. Therefore, the maturity (maturation state) of a cell can be determined by sequentially computing a characteristic (referred to hereinafter as the stratification characteristic) reflating to the stratification of cells between or at each time point from the time-lapse images to determine the temporal variation of the stratification characteristic.

In the present specification, (1) a statistic based on a degree of similarity by block matching of local regions between viewing images, (2) a statistic based on luminance values near the contour of a cell aggregation, and (3) a statistic based on the contour shape of a cell aggregation are presented as the stratification characteristic reflating to the stratification of cells.

### (1) Method Using a Degree of Similarity by Block Matching of Local Regions Between Images

By this method, for the viewing image (first image) from time point t and the viewing image (second image) from the next time point t+1 in time-lapse images taken in a predetermined time period, block matching of luminance distributions is performed for a neighbourhood that incudes the corresponding position of the cell in the second image using the luminance distribution of a local region of a cell in the first image as a template, the degree of similarity of the position domain having the highest degree of matching (the position domain in which the variation of the luminance distribution within the region is smallest) is taken as the representative degree of similarity of the position domain, and a statistic based on the representative degree of similarity is taken as the stratification characteristic.

This method takes advantage of the fact that an image having a stratified part and a single-layered part in which cells are not stratified has the hollowing features. For a single-layered cell aggregation in which a single cell grows or a plurality of cells congregates and spreads in the horizontal direction, with respect to an image from the adjacent time point, the boundaries between cells are observable even when there is movement or rotation of individual cells, and the internal structure of the cells is maintained. On the other hand, in a case in which stratification of cells occurs, since division or movement occurs in the vertical direction within the cell aggregation and changes occur such that bubbles form, the spatial structure or the brightness of the image significantly varies.

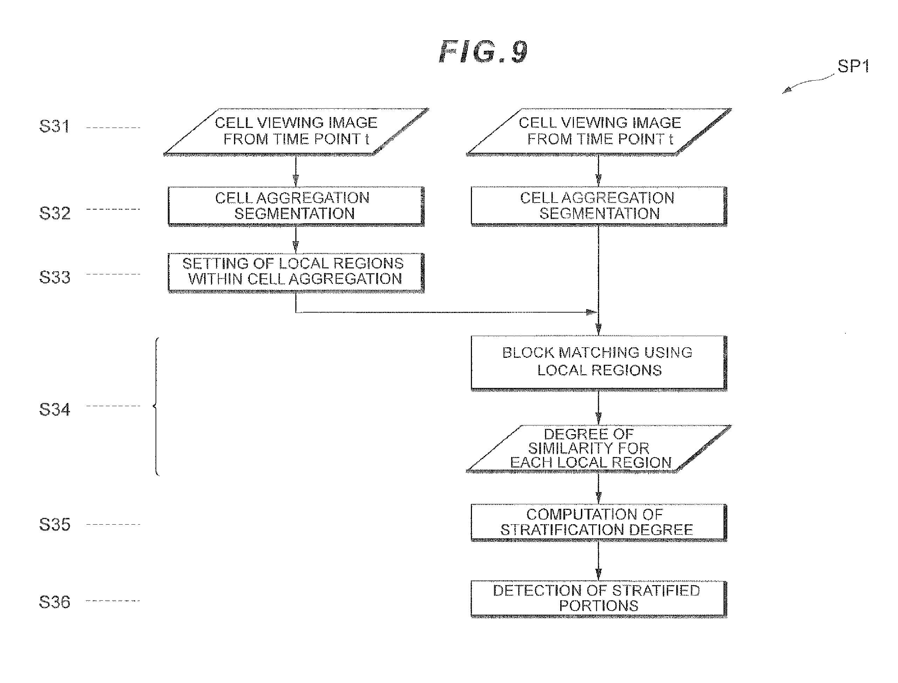

Therefore, since interval changes in a cell aggregation for single-layer regions are accounted for primarily by spatial movement, although there is a high degree of watching when block matching is performed in an area that includes corresponding positions in two images, changes within cells in a stratified region involve not only spatial movement but structural change as well, and the degree of matching decreases despite peripheral searching. A correlation value, a difference, a product, or another value may be used as an indicator for the degree of similarity, and in the case that a correlation value, for example, is used, the representative degree of similarity in a single-layer region is high, the representative degree of similarity in a stratified region is low, and the state of stratification can be determined by the size of the representative degree of similarity, Block matching of local regions between images is performed in the image processing device 100. The flowchart of FIG. 9 shows the portion SP1 of the image processing program GP that performs professing for detecting stratified sites in the present method.

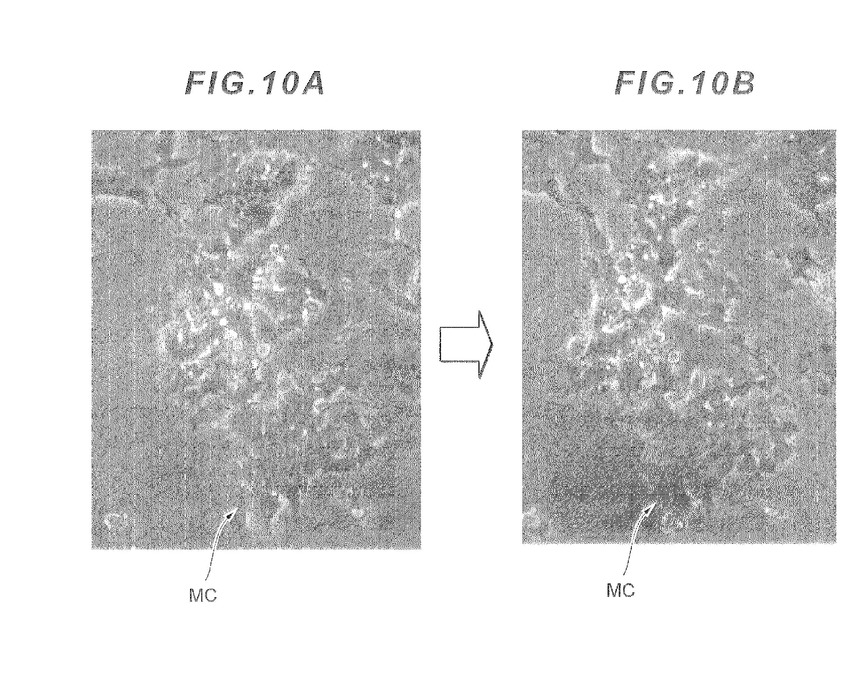

In the first step S31, the image analyzer 120 obtains the first image (the viewing image shown in FIG. 10A, for example) from time point t and the second image (the viewing image shown in FIG. 10B, for example) from the next time point t+1 from the time-lapse images stored in the RAM 63, and in step S32, the cells included in the viewing images are extracted and labeled, and the cells of the first image and the cells of the second image are associated by the same procedure as described above in the image professing programs GP1, GP2. At this time, the positions of the cells are aligned in order to reduce the effect of rotation or movement of cells between the images. Positional alignment is performed based on the center of mass of each cell, the coroner positions of bounding rectangles, or on another basis, and the effect of rotation is suppressed by lining up angles so that the correlation of shape moments is maximized (the difference is minimized).

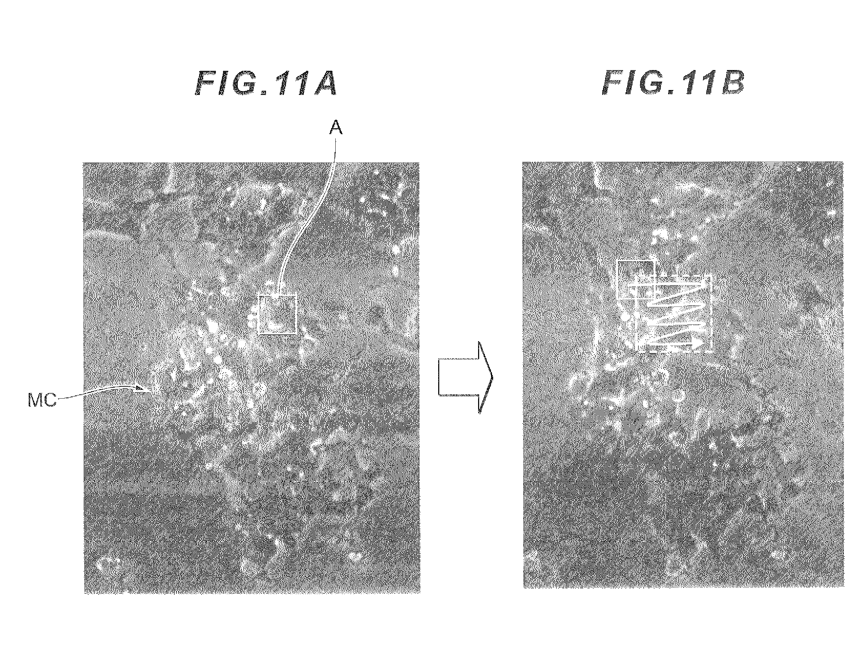

In the next step S33, a local region A centered at a pixel which forms the image is set for a cell of the first image. The "local region" A, as shown surrounded by an outline frame if FIG. 11A, is set so as to be adequately small in relation to the cell aggregation, and is set to about 5×5 to 15×15 (the size of two to three origin cells), for example. The position of the local region may be automatically set using a contour edge of an extracted cell as a start point.

Block matching is performed in step S34 for the local region thus set. Using the luminance distribution of the local region A set as shown in FIG. 11A in the first image as a reference, block matching is performed by scanning the luminance distribution of the local region A for the area that includes the corresponding position in the second image to compute a degree of similarity in each position, and searching for the most highly matching positions, as shown in FIG. 11B. A correlation value, a difference, a product, or another value of the luminance distribution may be used as an indicator for the degree of similarity. In the case that a correlation value is used, the position having the greatest (near 1) value is searched, and in the case that a difference is used, the position having the smallest (near 0) value is searched. The degree of similarity of the most highly matching position is stored in the RAM 63 as the representative degree of similarity. The following description is of a case in which a correlation value is used as the degree of similarity.

In the case that the local region is a single-layer structure, since changes in the cell aggregation over time are accounted for primarily by cell movement, the correlation value for the representative degree of similarity by block matching takes a large value (a correlation value near 1) in the area that includes the corresponding position. On the other hand, in the case that the local region is at a site of stratification, since changes in the cell aggregation over time involve deformation of the spatial structure or fluctuation of luminance, the correlation value of the representative degree of similarity takes a small value (near zero) despite area searching. In step S34, sequential block matching is performed while the local region A of the first image as the basis for comparison is moved by a predetermined number of pixels (one or more pixels) within the image, and a representative degree of similarity for each portion is computed, for the entire area of the cell aggregation. The representative degree of similarity of each portion obtained by this block matching indicates the state of stratification of the corresponding portion, and the distribution of representative degrees of similarity is used to indicate that state of stratification in the cell aggregation as a whole.

Here, the correlation value decreases in size as stratification progresses from a single-layer state, and the size of the correlation value is not convenient for vindicating the degree of stratification. The correlation value of the representative degree of similarity is therefore inserted in step S35 in the image processing device 100 so that the value increases (so as to approach 1 from 0) as stratification progresses. Specifically, the value of 1 minus the correlation value is taken when the correlation value is used as the degree of similarity, and the absolute value of the difference is taken when a difference value is used as the degree of similarity. In the present specification, the representative degree of similarity computed by this professing is referred to as the "stratification degree." The correlation may take values of -1 to +1 by calculation, but because a negative value (-1 to 0) indicates a case of inverted luminance, which has no meaning in terms of cell shape, negative computed correlation values are charged to zero, and the value of 1 minus the correlation value is taken.

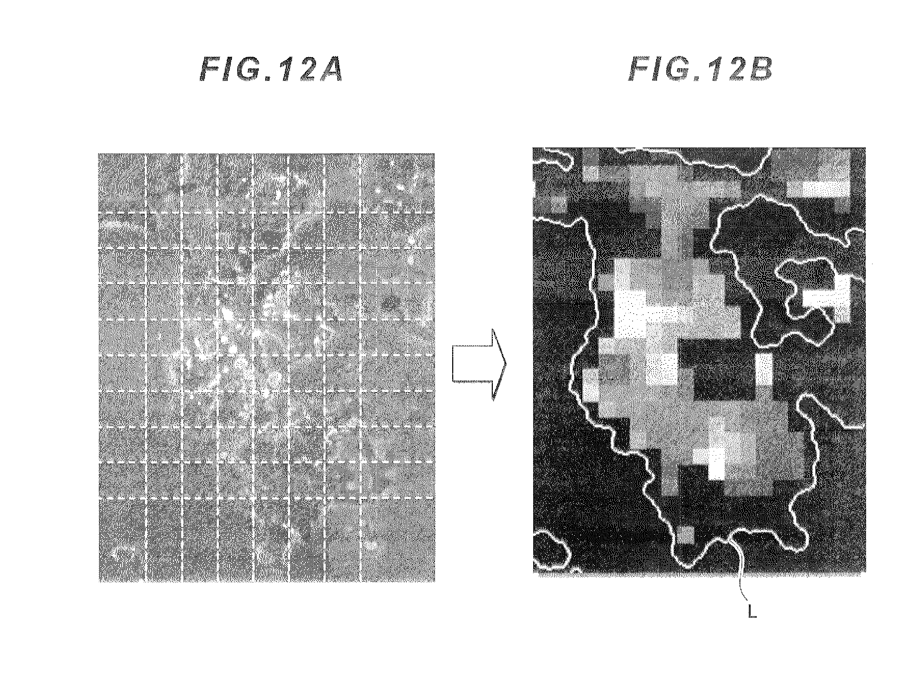

In FIG. 12, (a) shows an example in which the size of the local regions is indicated by dashed lines, and (b) shows an example in which the distribution of stratification degrees computed by step S35 is shown so as to be easy to determine visually. In (b) of FIG. 12, inside the cell aggregation MC surrounded by the contour shape line L, locations having a low stratification degree are shown dark, locations heaving a high stratification degree are shown bright, and multiple gradation levels are displayed corresponding to the degree of stratification. As is apparent from FIG. 12, the progress of stratification and the site at which stratification is occurring in a cell aggregation can be determined for each time point in the second image for each cell aggregation that is included in the viewing image.

The image professing program SP1 is also provided with a function for differentiating between stratified locations and non-stratified locations by using the stratification degree computed in step S35. Specifically, in step S36, the value of the stratification degree computed in step S35 and a predetermined threshold value set in advance are compared, and regions in which the stratification degree is equal to or greater than the threshold value are determined to have stratification. The processing step S36 is executed in accordance with the processing flow of the image processing program described below, or a display request or the like from an operator.

A statistic based on the degree of similarity is computed using the stratification degree of each portion of the cell aggregation for each time point, computed as described above, and the maturity of the cell aggregation is determined by the time-lapse variation of the statistic. The statistic based on the degree of similarity may be (i) a sum of stratification degrees or (ii) the occupancy ratio of stratified portions.

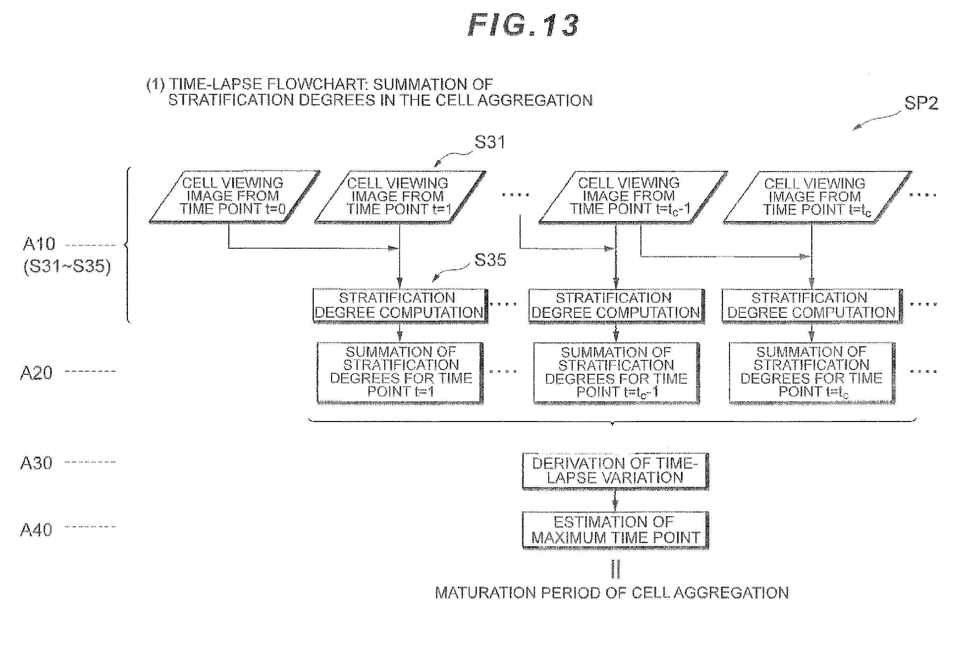

In the method using (i) the sum of the stratification degrees as the statistic which is based on the degree of similarity, the stratification degrees of the portions of the cell aggregation for each time point computed in step S35 are added together for each entire cell aggregation, and, the maturation state (maturity) of the cell aggregation is determined by calculating the summation of the stratification degrees of each time point for each cell aggregation and depriving the time-lapse variation of the total stratification degree. FIG. 13 is a flowchart including the program SP1 for detecting stratified portions described above, and shows an image professing program SP2 for determining the maturity of a cell aggregation on the basis of the time-lapse variation of the total stratification degree.

In the flowchart shown in FIG. 13, the program SP1 (S31 through S35) for detecting the stratified portions of a cell aggregation described above is included in step S10, and in this step A10, the stratification degree of each portion of the cell aggregation at time point t+1 is computed by block matching of local regions from the first image from time point t and the second image from time point t+1. For example, the stratification degree of each portion of the cell aggregation at time point t=1 is computed from the viewing image (first image) from time point t=0 and the viewing image (second image) from time point t=1, block matching of local regions with the viewing image from the next time point is performed in sequence in the same manner thereafter, and the stratification degree of each portion of the cell aggregation at time point t_{c} is computed from the viewing image (first image) from time point t=t_{c}-1 and the viewing image (second image) from time point t=t_{c}.

In step A20, the stratification degrees of the portions of the cell aggregation for each time point computed in step A10 (refer to FIG. 12B) are added together for each entire cell aggregation, and the summation of the stratification degrees of each time point (time point t=1, 2, ..., t_{c}-1, t_{c}) is computed for each cell aggregation. It is thereby possible to assess the degree to which each cell aggregation is stratified at each time point,

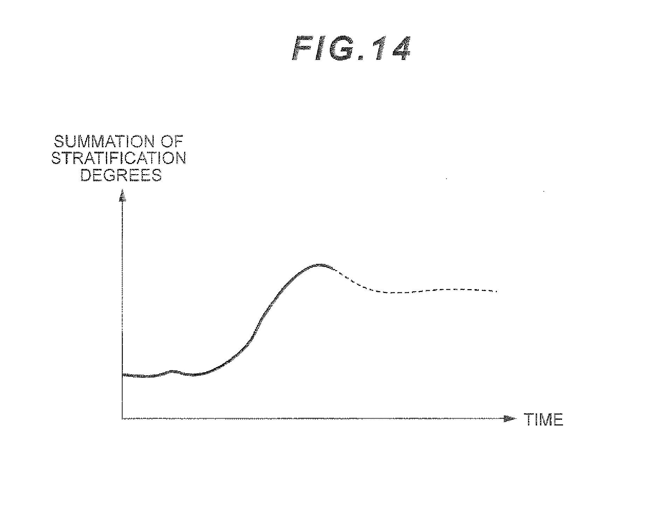

In step A30, the values of the total stratification degree for each time point computed in step A20 are arranged in a time lapse along the time points t=1, 2, 3, ..., t_{c}-1, t_{c}, and the time-lapse variation of the total stratification degree is derived for each cell aggregation. FIG. 14 is a graph of the temporal variation of the total stratification degree computed by the processing of step A20 for deriving the time-lapse variation, for a single cell aggregation in the viewing images. The horizontal axis in the graph is the elapsed time since time point t=0, and the vertical axis is the total stratification degree, which is a value from 0 to 1 when normalized by dividing the total value by the number of partitions of the local region.

In the initial stage of cell culturing, since the cells in the cell aggregation spread in only two dimensions and the structure of each cell is distinct, the total stratification degree remains at a small value. As the cell aggregation grows and stratification begins, the cell structure changes dramatically in three dimensions and increases in complexity, and the total stratification degree therefore begins to increase and continues to increase as the stratified region enlarges. When the stratified region expands to substantially the entire area of the cell aggregation, the increase in the total value slows until there is almost no increase thereof. As more time elapses, since the individual cells of the now stratified region become extremely small and structural changes occur on a small scale, the total stratification degree gradually decreases after reaching the maximum value thereof, and this trend continues.

The maturation state of each cell aggregation can therefore be determined from time-lapse variation information (referred to as stratification degree time-lapse information) of the total stratification degree derived by the processing of step A30. For example, it is possible to determine that growth of stratification has begun in the cell aggregation when the total stratification degree begins to increase, and it is possible to determine that growth is under way when the total stratification degree is increasing in the stratification degree time-lapse information. The cell aggregation can also be determined to be mature when the total stratification degree reaches a maximum value equal to or greater than a predetermined value, or when the total stratification degree enters a stable period or a decreasing period after crossing a peak.

In the image processing program SP2, a calculation is made in step A40 as to whether the total stratification degree has reached the maximum value thereof by a designated time point t_{c} (e.g., the nearest viewing timing) for each cell aggregation in the viewing image, from the time-lapse information of the stratification degree derived in step A30, and in the case that the maximum value has been reached, the cell aggregation is determined to be a mature cell aggregation. On the other hand, in the case that the maximum value has not been reached by the designated time point t_{c}, the cell aggregation is determined to be immature.

In the basis of the determination of maturation by the image processing program SP2, the analysis of cell configuration by the image processing programs GP1, GP2 described above is performed for the cell aggregation that is determined to be mature, and a display of cell information or origin cells is outputted. Through this configuration for analysts in which maturity is determined in advance and adequately mature cells are automatically selected for the designated time point, immature cells and the like which do not require analysis can be excluded to reduce the professing burden, and origin information can be analyzed at high speed only for adequately mature cells that are used for division, drug effect testing, and other procedures.

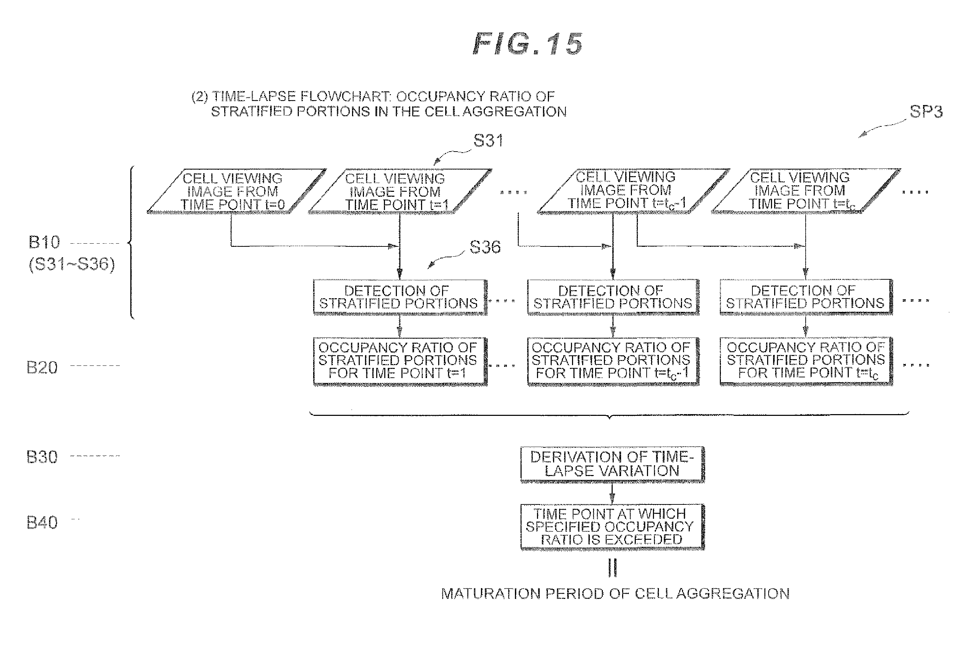

In the method using (ii) the occupancy ratio of stratified portions as the statistic which is based on the degree of similarity, the ratio occupied by stratified portions with respect to the entire cell aggregation (or a designated region in the case that a range for analysis is designated), i.e., the occupancy ratio of stratified portions, is computed in each cell aggregation for each time point from the distribution of the stratification degrees of the portions of the cell aggregation computed in step S35, and the maturation state (maturity) of the cell aggregation is determined by deriving a time-lapse variation of the occupancy ratio of stratified portions. FIG. 15 is a flowchart including the program SP1 for detecting stratified portions by block mapping described above, and shows an image processing program SP3 for determining the maturity of a cell aggregation on the basis of the time-lapse variation of the occupancy ratio of stratified portions.

In the flowchart of FIG. 15, the program SP1 (S31 through S36) for detecting stratified portions of a cell aggregation described above is included in step B10, and in this step B10, the stratification degree of each portion of the cell aggregation at time point t+1 is computed by block mapping of local regions from the first image from time point t and the second image from the next time point t+1, and a stratified portion is detected according to the size of the computed, stratification degree. For example, a stratified portion of the cell aggregation at time point t=1 is detected from the viewing image (first image) from time point t=0 and the viewing image (second image) from time point t=1, block matching with the viewing image from the next time point is performed in sequence in the same manner thereafter, and the stratified portion of the cell aggregation at time point t_{c} is detected from the viewing image (first image) from time point t=t_{c}-1 and the viewing image (second image) from time point t=t_{c}.

In step B20, the ratio of area occupied (area ratio) by stratified portions in each cell aggregation is computed for stratified portions detected in step B10, and the occupancy ratio of stratified portions at each time point (time t=1, 2, ..., t_{c}-1, t_{c}) is computed for each cell aggregation. It is thereby possible to assess the degree to which each cell aggregation is stratified at each time point.

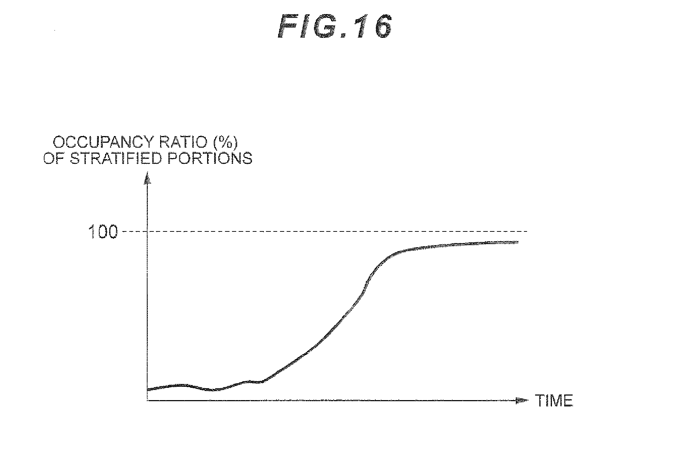

In step B30, the values of the occupancy ratio of stratified portions for each time point computed in step B20 are arranged in a time lapse along the time points t=1, 2, 3, ..., t_{c}-1, t_{c}, and the time-lapse variation of the occupancy ratio of stratified portions is derived for each cell aggregation. FIG. 16 is a graph of the temporal variation of the occupancy ratio of stratified portions computed by the processing of step B20 for deriving the time-lapse variation, for a single cell aggregation in the viewing images. The horizontal axis in the graph is the elapsed time since time point t=0, and the vertical axis is the occupancy ratio of stratified portions, which is a value from 0 to 100%.

In the initial stage of cell culturing, since there are almost no stratified portions, the occupancy ratio of stratified portions remains low. As the cell aggregation grows and stratification begins, the occupancy ratio begins to increase, and increases as the stratified region enlargers. When the stratified region expands to substantially the entire area of the cell aggregation, the increase in the total value slows until there is almost no increase thereof.

The maturation state of each cell aggregation can therefore be determined from time-lapse variation information (referred to as stratification occupancy time-lapse information) of the occupancy ratio of stratified portions derived by the processing of step B30. For example, it is possible to determine that growth of stratification has begun in the cell aggregation when the occupancy ratio of stratified portions begins to increase, and it is possible to determine that growth is under way when the occupancy ratio of stratified portions is increasing in the stratification occupancy time-lapse information. The cell aggregation can also be determined to be mature when the occupancy ratio of stratified portions is equal to or greater than a predetermined value.

In the image processing programs SP3, the occupancy ratio of stratified portions at the designated time point t_{c} (e.g., the nearest viewing timing) and a specified occupancy ratio set in advance as a reference for determining maturity are compared in step B40 for each cell aggregation in the viewing image from the stratification occupancy time-lapse information derived in step B30, and the cell aggregation is determined to be a mature cell aggregation in the case that the occupancy ratio of stratified portions is equal to or greater than the specified occupancy ratio. In the case that the occupancy ratio of stratified portions at the specified time point t_{c} is less than the specified occupancy ratio, the cell aggregation is determined to be immature. The specified occupancy ratio is set as appropriate according to the type or characteristics of the cells being viewed, the purpose for viewing, and other factors, but is generally set within the range of about 70 to 90% in the case of iPS cells, ES cells, and the like.

On the basis of the determination of maturation by the image processing program SP3, the analysis of cell configuration by the image processing programs GP1, GP2 described above is performed for the cell aggregation that is determined to be mature, and a display of cell information or origin cells is outputted. Through this configuration for analysis in which maturity is determined in advance and adequately mature cells are automatically selected for the designated time point, immature cells and the like which do not require analysis can be excluded to reduce the processing burden, and origin information can be analyzed at high speed only for adequately mature cells that are used for division, drug effect testing, and other procedures. A maturation determination method using "a statistic based on luminance values near the contour of a cell aggregation" as the stratification characteristic will next be described.

### (2) Method Using a Statistic Based on Luminance Values Near the Contour of a Cell Aggregation

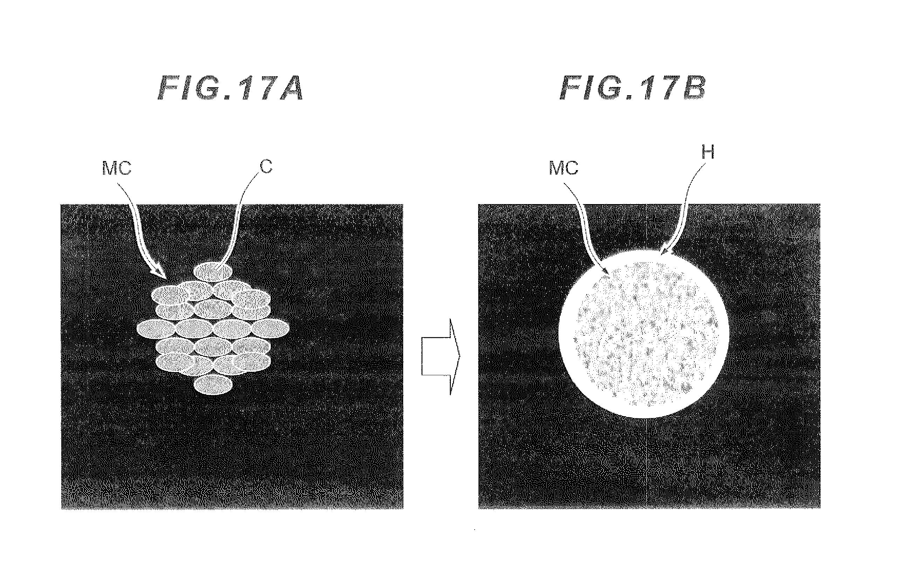

FIG. 17 is a schematic view showing a viewing image taken during plate culturing of cells. At the start of culturing, congregated cells C adhere to the Petri dish or other medium surface, and a cell aggregation spreads in two dimensions, as shown in FIG. 17A. There is therefore no significant "halo" near the contour of the cell aggregation MC. However, when the cell aggregation grows and stratifies so as to proliferate in three dimensions, a thickness occurs at the contour of the cell aggregation, and a halo H occurs near the contour of the cell aggregation MC, as shown in FIG. 17B. The halo near the contour is more clearly evident when the viewing optical systems (54a, 55a) are phase-contrast microscopes.

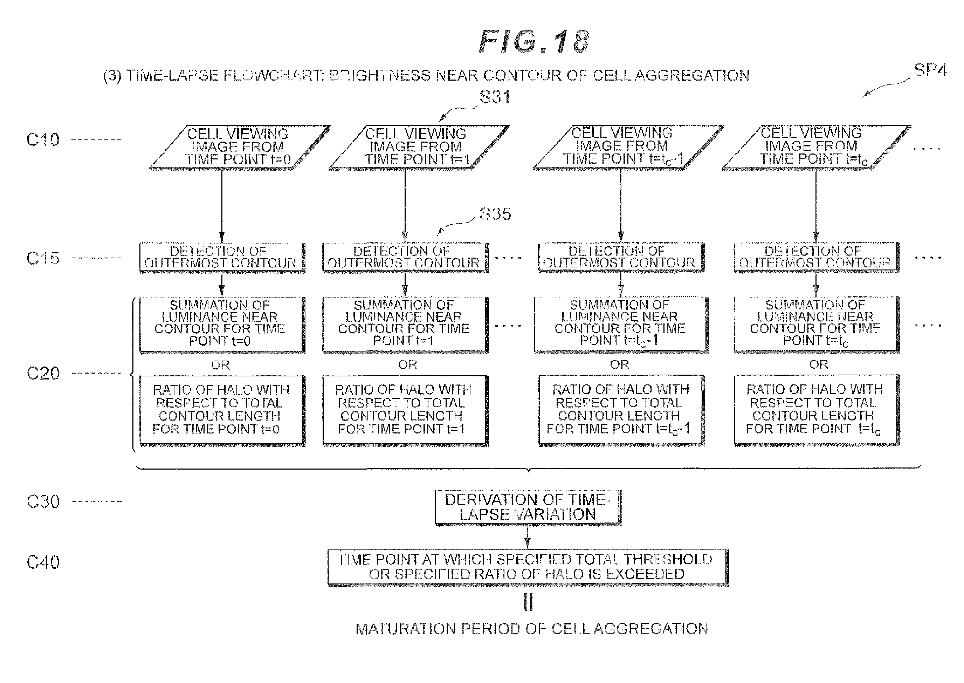

The present method makes use of the fact that a halo forms near the contour and luminance values vary as the cell aggregation stratifies and matures. Examples of statistics based on luminance values include the luminance summation obtained by adding together the luminance values near the contour of the cell aggregation, and the ratio (Halo length/Total contour length) of the length of the portion (halo) of the contour having a luminance equal to or greater than a certain luminance value with respect to the total contour length of the cell aggregation. FIG. 18 is a flowchart showing the image processing program SP4 of the present method.

In the first step C10, the image analyzer 120 reads the time-lapse images for time points t=0, 1, 2, ..., t_{c}-1, t_{c} stored in the RAM 63, and in step C15, extracts and labels the cells included in the viewing images and associates cells between images by the same method as described in the image processing programs GP1, GP2 for the viewing images from each time point.

In step C20, the statistic based on luminance values described above is computed for the contour portion of a cell aggregation for each time point extracted in step C15. For example, in the case that the statistic based on luminance values is the summation of luminance near the contour, the summation of the luminance values in the region adjacent to the contour of each cell aggregation is computed for each cell aggregation. The size of the adjacent region, i.e., the width of the border in the outer circumferential direction with respect to the contour line extracted in step C15, is set so as to be appropriate for the region in which the halo observed by the viewing system appears when the viewed cell aggregation is stratified. In the case that the statistic based on luminance values is the ratio of the length of the halo to the total contour length of the cell aggregation, the computed value is the ratio of the length in the direction along the contour line of the portion having a luminance equal to or greater than a certain luminance value in the region adjacent to the cell aggregation with respect to the total contour length of the cell aggregation extracted in step C15.

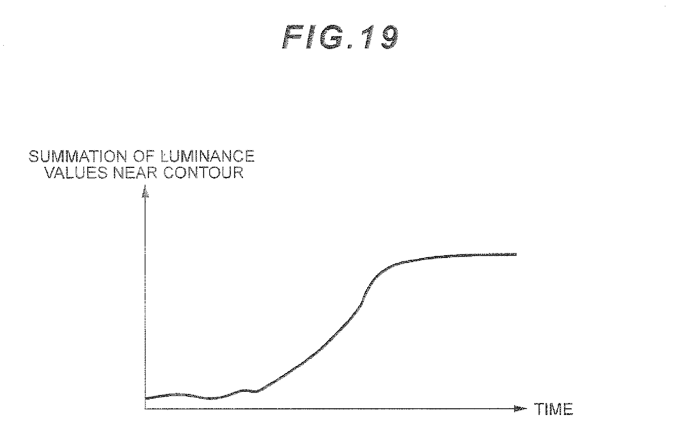

In step C30, the statistic based on luminance values for each time point extracted in step C20 is arranged in a time-lapse along the time points t=0, 1, 2, ..., t_{c}-1, t_{c}, and the time-lapse variation of the statistic based on luminance values is derived for each cell aggregation. FIG. 19 is a graph of the temporal variation of the summation of luminance near the contour computed by the processing of step C20 for deriving the time-lapse variation, for a single cell aggregation in the viewing images. The horizontal axis in the graph is the elapsed time since time point t=0, and the vertical axis is the summation of the luminance values near the contour.

As shown schematically in FIG. 17, in the initial stage (a) of cell culturing, since the congregated cells C adhere to the medium surface and spread in two dimensions almost no haloing occurs around individual cells C or near the contour of the congregated cell aggregation MC, and the summation of luminance near the contour varies in a low state. As the cell aggregation grows and stratification begins, a bright halo appears near the contour in the neighbourhood of the portion where the thickness is increased by stratification, the luminance summation begins to gradually increase, and the luminance summation increases as the stratified region enlarges. When the stratified region expands to fill substantially the entire area of the cell aggregation MC as shown in FIG. 17B, the entire cell aggregation is surrounded by a bright halo H, and the increase in the summation of luminance near the contour substantially stops. The progress of the time-lapse variation is the same in the case that the ratio of the length of the halo with respect to the total contour length of the cell aggregation is used as the statistic based on luminance values.

Consequently, the maturation state of each cell aggregation can be determined from the time-lapse variation information (referred to as the luminance statistic time-lapse information) of the statistic based on luminance values that is extracted by the processing of step C30. For example, it is possible to determine that growth of stratification has begun in the cell aggregation when the summation of luminance near the contour begins to increase, and it is possible to determine that growth is under way when the luminance summation is increasing in the luminance statistic time-lapse information. The cell aggregation can also be determined to be mature when the increasing trend of the luminance summation becomes more moderate and the rate of increase is equal to or less than a predetermined value, or when the luminance summation is equal to or greater than a specified summation threshold.

In the image processing program SP4, the summation of luminance near the contour at the designated time point t_{c} (e.g., the nearest viewing timing) and a specified summation threshold set in advance as a reference for determining maturity are compared in step C40 for each cell aggregation in the viewing image from the luminance statistic time-lapse information derived in step C30, and the cell aggregation is determined to be a mature cell aggregation in the case that the summation of luminance near the contour is equal to or greater than the specified summation threshold. In the case that the summation of luminance near the contour at the specified time point t_{c} is less than the specified summation threshold, the cell aggregation is determined to be immature. In the same manner in the case that the statistic based on luminance values is the ratio of the halo length with respect to the total contour length of the cell aggregation, the ratio of the halo length with respect to the total contour length of the cell aggregation at the designated time point t_{c} and the specified ratio of the halo set in advance as a reference for determining maturity are compared, and the cell aggregation is determined to be mature when the ratio of the halo length with respect to the total contour length is equal to or greater than the specified ratio.

On the basis of the determination of maturation by the image processing program SP4, the analysis of cell configuration by the image processing programs GP1, GP2 described above is performed for the cell aggregation that is determined to be mature, and a display of cell information or origin cells is outputted. Consequently, in this method as well, since cells that are adequately mature at the designated time point are automatically selected and the origin cells and the like thereof are analyzed, immature cells and the like which do not require analysis can be excluded to reduce the processing burden, and origin information can be analyzed at high speed. A maturation determination method using "a statistic based on the contour shape of a cell aggregation" as the stratification characteristic will next be described.

### (3) Method Using a Statistic Based on the Contour Shape of a Cell Aggregation

As shown in FIG. 17A, in the initial stage of cell culturing, cells C congregate to form a cell aggregation MC, and individual cells are therefore present near the contour of the two-dimensionally spreading cell aggregation MC, and the contour of the cell aggregation has a complex shape including numerous projections and depressions. As growth of the cell aggregation progresses, the projections and depressions of the contour portion are gradually absorbed, and the contour shape becomes smooth. By the time that a three-dimensional cell structure is formed by stratification, the contour shape of the cell aggregation MC has become relatively round, as shown in FIG. 17B.

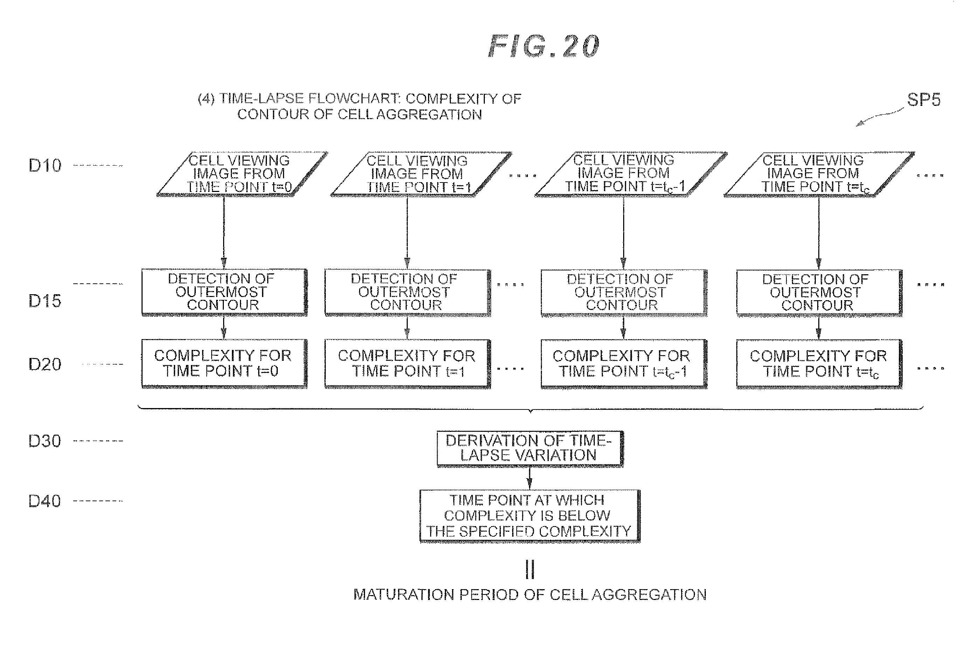

The present method thus takes advantage of the fact that the contour shape of the cell aggregation varies as the cell aggregation matures. The complexity of the contour of the cell aggregation is presented as a typical example of a statistic which is based on the contour shape of the cell aggregation. The complexity of the contour of a cell aggregation can be specified by, for example, the ratio of the circumferential length with respect to the area of the cell aggregation (Circumferential length/Area). FIG. 20 is a flowchart showing the image processing program GP5 of the present method.

In step D10, the image analyser 120 reads the time-lapse images for time points t=0, 1, 2, ... , t_{c}-1, t_{c}, and in step D15, extracts and labels the cells included in the viewing images and associates cells between images by the same method as described in the image processing programs GP1, GP2 for the viewing images from each time point.

In step D20, the statistic based on the contour shape of the cell aggregation described above is computed for the contour portion of a cell aggregation for each time point extracted in step D15. In the present example, the ratio of the circumferential length (total contour length) with respect to the area of a cell aggregation for which the outermost contour portion is extracted is computed to obtain the complexity of the contour of the cell aggregation.

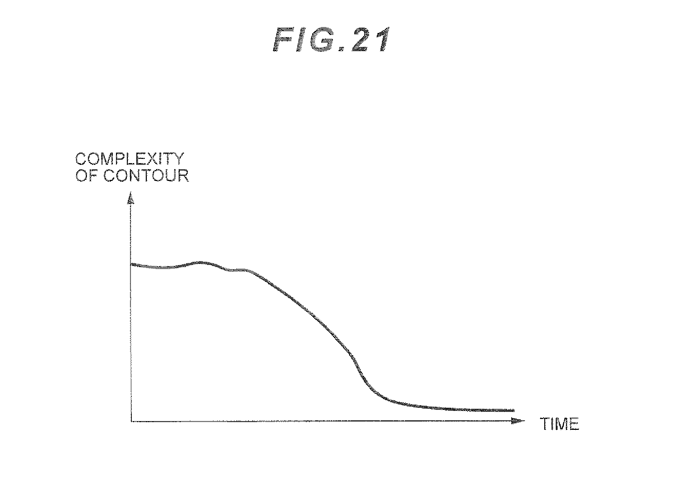

In step D30, the statistic based on contour shape for each time point computed in step D20 is arranged in a time-lapse along the time points t=0, 1, 2, ..., t_{c}-1, t_{c}, and the time-lapse variation of the statistic based on contour shape is derived for each cell aggregation. FIG. 21 is a graph of the temporal variation of the complexity of the cell aggregation contour computed by the processing of step D20 for deriving the time-lapse variation, for a single cell aggregation in the viewing images. The horizontal axis in the graph is the elapsed time since time point t=0, and the vertical axis is the complexity of the contour.

As shown in FIGS. 17A and 17B, in the initial stage of cell culturing, since cells congregate so that a cell aggregation spreads in two dimensions, and the contour of the cell aggregation has a complex shape including numerous projections and depressions, the complexity of the contour computed in step D20 fluctuates at a high value. As growth of the cell aggregation progresses, the projections and depressions of the contour portion are gradually absorbed, the contour shape becomes smooth, and the complexity of the contour decreases over time. As stratification within the cell aggregation progresses further, the contour shape of the cell aggregation becomes circular or round, and the low complexity value of the contour substantially ceases to decrease.

Therefore, the maturation state of each cell aggregation can be determined from the time-lapse variation information (referred to as the contour shape statistic time-lapse information) of the statistic based on contour shape of the cell aggregation that is extracted by the processing of step D30. For example, it is possible to determine that a period of transition to stratification is under way in the cell aggregation when the complexity of the contour begins to decrease, and it is possible to determine that growth of stratification is under way when the complexity of the contour is degreasing in the contour shape statistic time-lapse information. The cell aggregation can also be determined to be mature when the decreasing trend of the complexity of the contour becomes more moderate and the rate of decrease is equal to or less than a predetermined value, or when the complexity of the contour is equal to or less than a specified complexity.

In the image processing program SP5 shown in FIG. 20, the complexity of the contour at the designated time point t_{c} (e.g., the nearest viewing timing) and a specified complexity set in advance as a reverence for determining maturity are compared in step D40 for each cell aggregation in the viewing image from the contour shape statistic time-lapse information derived in step D30, and the cell aggregation is determined to be a mature cell aggregation in the case that the complexity of the contour of the cell aggregation is equal to or less than the specified complexity. In the case that the complexity of the contour of the cell aggregation at the specified time point t_{c} exceeds the specified complexity, the cell aggregation is determined to be immature.

On the basis of the determination of maturation by the image processing program SP5, the analysis of cell configuration by the image processing programs GP1, GP2 described above is performed for the cell aggregation that is determined to be mature, and a display of cell information or origin cells is outputted. Consequently, in this method as well, since cells that are adequately mature at the designated time point are automatically selected and the origin cells and the like thereof are analyzed, immature cells and the like which do not require analysis can be excluded to reduce the processing burden, and origin information can be analyzed at high speed.

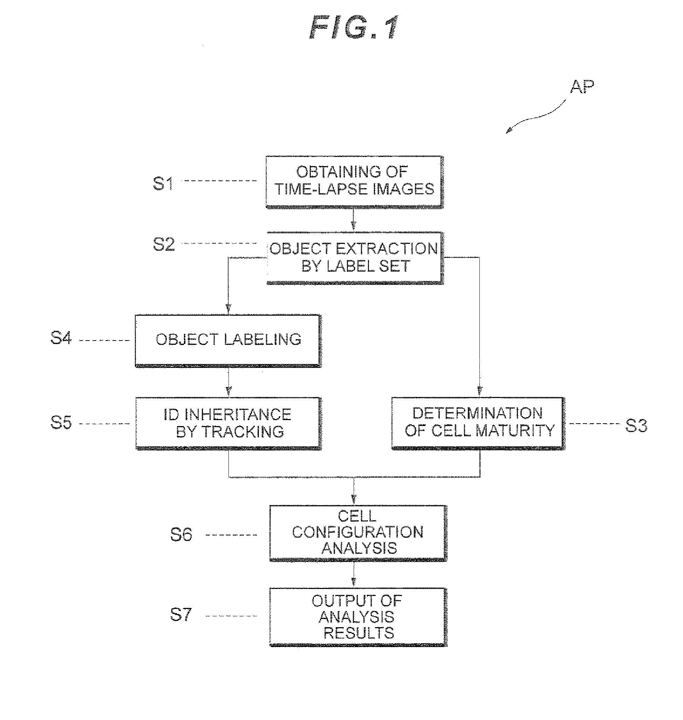

FIG. 1 is a rough flowchart showing an automated analysis program AP for automatically selecting cells that are adequately mature at a specified time point t_{c} and analyzing the cells for origin cells and the like by combining the image processing programs GP (GP1, GP2) and the image processing programs SP (SP1 through SP5) described above.

In the automated analysis program AP, the time-lapse images for the time points t=0, 1, 2, ... , t_{c}-1, t_{c} are first read and obtained in step S1, and in step S2, extraction and labeling of the cells included in the viewing images, and association of cells between images are performed by the method described above for the viewing images from each time point.

Then, in step S3, maturity determination for determining whether each cell aggregation is adequately mature is performed for each cell aggregation included in the viewing image at the designated time point t_{c} (e.g., the nearest viewing timing), by any of the image processing programs SP1 through SP5 or a combination of the image processing programs SP1 through SP5.

In step S4, ID assignment to relevant cell aggregations is performed for cell aggregations that are determined to be mature by the maturity determination of step S3 (or an ID is assigned to all cell aggregations except for those that are determined to be immature). Then, ID inheritance (inheritance of cell information) by cell tracking according to at least one of the image processing programs GP1, GP2, i.e., of the time-lapse images in one or both the backward direction and the forward direction of the time axis, is performed in step S5. In step S6, classification, computation of the number of constituent cells and the origin cells of the cells, and other analysis is performed, and the results of analysis are outputted to the display panel 72 or elsewhere in step S7.

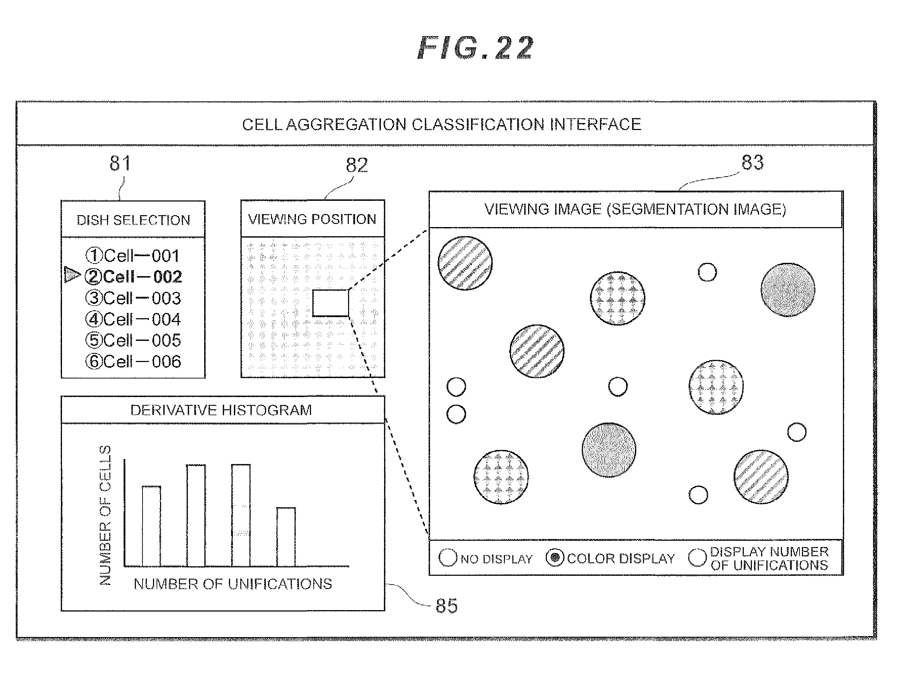

FIG. 22 shows an example of the configuration of a user interface of an application for executing the automated analysis program AP and displaying the analysis results. In the cell aggregation classification interface of this example, the screen of the display panel 72 is provided with a dish selection frame 81 for selecting a viewing subject from the plurality of cell culture containers 10 accommodated in the cell culture chamber 2; a viewing position display frame 82 for designating a specific viewing position from the full image of the selected cell culture container; a viewing image display frame 83 for displaying an image of the analysis results for the designated viewing position; a derivative histogram display frame 85 for displaying a histogram corresponding to the number of origin cells for a cell aggregation included at the designated viewing position (or in the full image) ; and other components.

The example shown is of a state in which the cell culture container having the code number Cell-002 is selected in the dish selection frame 81, and the area enclosed by a border in the viewing position display frame 82 is designated using a mouse or the like. Analysis results for a cell aggregation that is determined to be mature at the designated time point t_{c} by the processing for determining mature cells is displayed in the viewing image display frame 83, and in the configuration shown in FIG. 22, a display is created that is color coded according to the number of origin cells constituting the cell aggregation. From this display, it is possible to instantly make accurate assessments of mature cell aggregations in the viewing image, such as whether a colony is present that is composed of a single cell or whether a cell aggregation is composed of several origin cells. By switching a display selection button provided at the bottom of the frame, the number of unification of each cell aggregation, the origin cells of each cell aggregation shown in FIG. 6, or other information can be displayed such as shown in Fig. 8, for example.

For cell aggregation that are determined to be mature at the designated time point t_{c}, the derivative histogram display frame 85 displays a histogram in which the horizontal axis represents the number of origin cells (number of unifications) constituting the cell aggregation, and the vertical axis represents the number of cell aggregations for each number of unifications. This histogram display makes it possible to obtain an overview of the cultured cell aggregations, and is useful information for evaluating data cells, cell culture conditions, and the like.

As described above, in the cell classification method, image processing program, and image processing device of the present intention, cells are extracted and associated from time-lapse images taken by an imaging device while first and second images are sequentially shifted along the time axis, cell unification or division is sequentially inherited as cell information of each cell, and cells are classified on the basis of the inherited cell information. Consequently, through the present invention, the origin and configuration of each cell can be accurately assessed and cells can be precisely selected and evaluated even in a case in which cell culture observation has progressed for a certain amount of time and numerous cells have reached maturity through growth or unification.

In the embodiment described above, an example is described of a configuration in which time-lapse images (image data) taken by the imaging device in the cell culture viewing system BS and stored in the RAM 63 are read to analyze configurations of cells, but a configuration may be adopted in which time-lapse images taken in another viewing system and recorded in a magnetic recording medium or the like, or time-lapse images transmitted via a communication line are read to analyze a cell configuration.

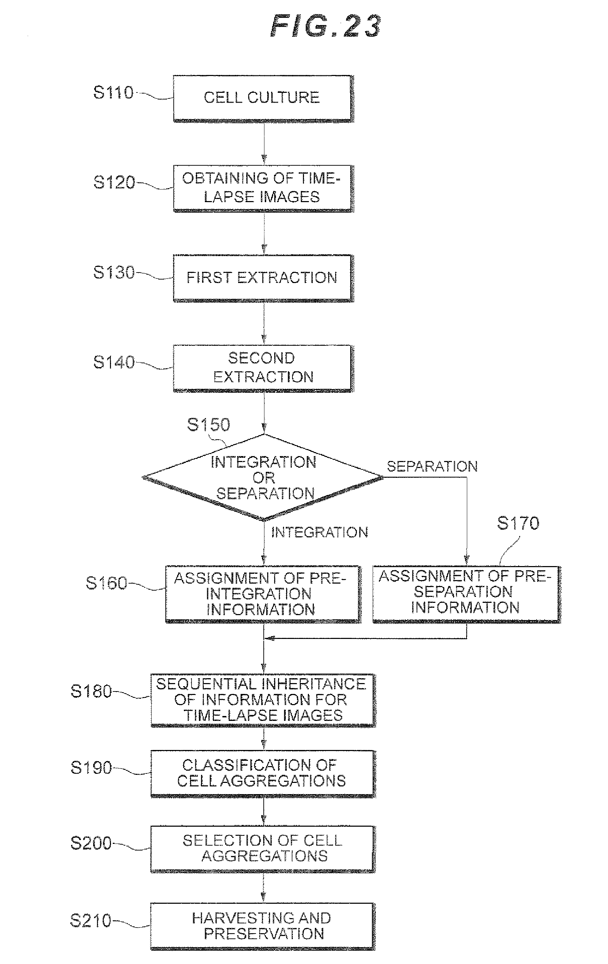

The method for producing a cell aggregation according to an embodiment of the present invention will next be described with reference to FIG. 23. This production method basically comprises a cell culture step (S110) of culturing cells, and a classification step (S120 through S190) of viewing, using the image processing device described above, the cells cultured in the cell culture step, and classifying cell aggregations of cells changed by cell culturing.

More specifically, the production method comprises a cell culture step (S110) of culturing cells; an obtaining step (S120) of photographing, through use of an imaging device, the cells cultured in the cell culture step and obtaining time-lapse images of cell aggregations of cells changed by cell culturing; a first extraction step (S130) of extracting cells included in a first image taken at a predetermined time point among the time-lapse images obtained in the obtaining step; a second extraction step (S140) of extracting cells included in a second image taken a predetermined time apart from the predetermined time point; a step (S160) of associating the cells extracted from the first image and the cells extracted from the second image, determining (S150) whether a plurality of cells of the first image is integrated in the second image, or whether a single cell of the first image is separated into a plurality of cells in the second image, and assigning pre-integration cell information to the integrated cell in the case that a plurality of cells of the first image is integrated into a single cell in the second image; and a step (S170) of assigning pre-separation cell information to the separated cells in the case that a single cell of the first image is separated into a plurality of cells in the second image. The production method further comprises an inheritance step (S180) of performing steps S130 through S170 for the time-lapse images obtained in step S120, executing the extraction and association of cells while sequentially allocating the time-lapse images as the first and second images along the time axis, and causing the cell information of the cells included in the images to be sequentially inherited; a classification step (S190) of classifying cell aggregations on the basis of the inherited cell information for the cells included in an image taken at an arbitrary time point; a selection step (S200) of selecting a cell aggregation on the basis of a predetermined reference; and a harvesting and preservation step (S210) of harvesting and preserving the selected cell aggregation. The cultured cells may be human, bovine, equine, porcine, murine, or other animal-derived cells, or may be plant-derived cells. Cell aggregations may also be preserved by cryopreservation.

### EXPLANATION OF NUMERALS AND CHARACTERS

- A:: local region
- BS:: cell culture viewing system
- C:: cell
- MC:: cell aggregation
- GP1, GP2:: image processing program
- SP1 through SP5:: image processing program (sub- programs for determining maturity)
- 5:: viewing unit
- 6:: control unit
- 54:: macro viewing system
- 54c:: imaging device
- 55:: micro viewing system
- 55c:: imaging device
- 61:: CPU (computer)
- 62:: ROM
- 63:: RAM
- 100:: image processing device
- 120:: image analyzer
- 130:: output unit

## Claims

1. A cell classification method comprising the steps of:
extracting cells included in the image from a first image taken at a predetermined time point;
extracting cells included in the image from a second image taken a predetermined time apart from the predetermined time point;
associating the cells extracted from the first image and the cells extracted from the second image, assigning pre-integration cell information to an integrated cell in the case that a plurality of cells of the first image is unified in the second image, and assigning pre-separation cell information to each separated cell in the case that a single cell in the first image is separated into a plurality of cells in the second image;
executing the extraction and association of cells while sequentially shifting the first image and the second image along a time axis for time-lapse images, and causing the cell information of the cells included in the images to be sequentially inherited; and
classifying cells on the basis of the inherited cell information for the cells included in an image taken at an arbitrary time point.

2. The cell classification method according to claim 1, **characterized in that**
inheritance of the cell information is executed in the backward direction of time to the start time of viewing, along the time axis of the time-lapse images, the predetermined time point at which the first image is taken being a time point t, and the time point at which the second image is taken being a time point t-1 the predetermined time prior to the predetermined time point; and
the cells in the image taken at the arbitrary time point are classified according to the number of cells that are origin cells constituting each cell, on the basis of the cell information of each cell inherited back to the start time of viewing.

3. The cell aggregation classification method based on cell configuration information according to claim 1, **characterized in that**
inheritance of the cell information is executed in the forward direction of time from the start time of viewing, along the time axis of the time-lapse images, the predetermined time point at which the first image is taken being a time point t, and the time point at which the second image is taken being a time point t+1 the predetermined time after the predetermined time point; and
the cells included in a viewing image taken at the arbitrary time point are classified according to the number of cells that are origin cells constituting each cell, on the basis of the cell information of each cell inherited up to the time point.

4. An image processing program for causing a computer to function as an image processing device for obtaining an image in which cells are photographed by an imaging device and performing image processing, the image processing program being readable by the computer; the image processing program comprising:
a first step of obtaining a first image taken at a predetermined time point by the imaging device and extracting cells included in the image;
a second step of obtaining a second image taken a predetermined time apart from the predetermined time point by the imaging device and extracting cells included in the image;
a third step of associating the cells extracted from the first image and the cells extracted from the second image, assigning pre-integration cell information to the integrated cell in the case that a plurality of cells of the first image is integrated into a single cell in the second image, and assigning pre-separation cell information to the separated cells in the case that a single cell of the first image is separated into a plurality of cells in the second image;
a step of executing the first through third steps for time-lapse images while sequentially shifting the first through third steps along the time axis, and causing the cell information of the cells included in each image to be sequentially inherited; and
a step of outputting the inherited cell information for cells included in an image taken at a designated time point.

5. The image processing program according to claim 4, further comprising a step of classifying, on the basis of the inherited cell information, the cells included in the image taken at the designated time point; and a step of outputting a classification result.

6. The image processing program according to claim 5, **characterized in that**
the step of inheritance of the cell information is executed in the backward direction of time to the start time of viewing, along the time axis of the time-lapse images, the predetermined time point at which the first image is taken being a time point t, and the time point at which the second image is taken being a time point t-1 the predetermined time prior to the predetermined time point; and
in the step of classifying the cells, cells in the image taken at the designated time point are classified according to the number of cells that are origin cells constituting each cell, on the basis of the cell information of each cell inherited back to the start time of viewing.

7. The image processing program according to claim 5, **characterized in that**
the step of inheritance of the cell information is executed in the forward direction of time from the start time of viewing, along the time axis of the time-lapse images, the predetermined time point at which the first image is taken being a time point t, and the time point at which the second image is taken being a time point t+1 the predetermined time after the predetermined time point; and
in the step of classifying the cells, the cells included in the image taken at the designated time point are classified according to the number of cells that are origin cells constituting each cell, on the basis of the cell information of each cell inherited up to the designated time point.

8. The image processing program according to any of claims 5 through 7, comprising:
a maturity determination step of computing a characteristic relating to stratification of cells from the first image and the second image sequentially obtained, and determining the maturity of each cell in an image at the designated time point on the basis of a time-lapse variation of the computed characteristic relating to stratification; wherein
cells determined to be mature in the maturity determination step are classified in the step of classifying the cells.

9. The image processing program according to claim 8, **characterized in that** the characteristic relating to stratification is a statistic based on a degree of similarity of a position domain of highest matching, the statistic being computed by talking as a template the luminance distribution of a local region of a cell aggregation in one image from the prior time point among the first image and second image of the time-lapse images, and performing block matching of luminance distributions for a neighbourhood that includes the corresponding position of the cell aggregation in the other image from the later time point.

10. The image processing program according to claim 8, **characterized in that** the characteristic relating to stratification is a statistic based on luminance values near a contour of the cell aggregation.

11. The image processing program according to claim 8, **characterized in that** the characteristic relating to stratification is a statistic based on contour shape of the cell aggregation.

12. An image processing device comprising an image analyzer for obtaining time-lapse images in which cells are photographed at a predetermined time interval by an imaging device; and an output unit for outputting results of analysis by the image analyzer;
the image analyzer extracting cells included in the image from a first image taken at a predetermined time point; extracting cells included in the image from a second image taken a predetermined time apart from the predetermined time point; associating the cells extracted from the first image and the cells extracted from the second image, assigning pre-integration cell information to an integrated cell in the case that a plurality of cells of the first image is unified in the second image, and assigning pre-separation cell information to each separated cell in the case that a single cell in the first image is separated into a plurality of cells in the second image; executing the extraction and association of cells while sequentially shifting the first image and the second image along a time axis for time-lapse images, and causing the cell information of the cells included in the images to be sequentially inherited; and
the output unit outputting the inherited cell information for the cells included in an image taken at a designated time point.

13. The image processing device according to claim 12, **characterized in that**
the image analyzer classifies the cells included in the image taken at the designated time point on the basis of the inherited cell information; and
the output unit outputs the results of classification by the image analyzer.

14. The image processing device according to claim 13, **characterized in that**
inheritance of the cell information is executed in the backward direction of time to the start time of viewing, along the time axis of the time-lapse images, the predetermined time point at which the first image is taken being a time point t, and the time point at which the second image is taken being a time point t-1 the predetermined time prior to the predetermined time point; and
in classification of the cells, cells in the image taken at the designated time point are classified according to the number of cells that are origin cells constituting each cell, on the basis of the cell information of each cell inherited back to the start time of viewing.

15. The image processing device according to claim 13, **characterized in that**
inheritance of the cell information is executed in the forward direction of time from the start time of viewing, along the time axis of the time-lapse images, the predetermined time point at which the first image is taken being a time point t, and the time point at which the second image is taken being a time point t+1 the predetermined time after the predetermined time point; and
in classification of the cells, the cells in the image taken at the designated time point are classified according to the number of cells that are origin cells constituting each cell, on the basis of the cell information of each cell inherited up to the designated time point.

16. The image processing device according to any of claims 13 through 15, **characterized in that** the image analyzer computes a characteristic relating to stratification of cells from the first image and the second image sequentially obtained, determines the maturity of each cell in an image at the designated time point on the basis of a time-lapse variation of the computed characteristic relating to stratification, and performs the classification for cells that are determined to be mature.

17. The image processing device according to claim 16, **characterized in that** the characteristic relating to stratification is a statistic based on a degree of similarity of a position domain of highest matching, the statistic being computed by taking as a template the luminance distribution of a local region of a cell aggregation in one image from the prior time point among the first image and second image of the time-lapse images, and performing block matching of luminance distributions for a neighbourhood that includes the corresponding position of the cell aggregation in the other image from the later time point.

18. The image processing device according to claim 16, **characterized in that** the characteristic relating to stratification is a statistic based on luminance values near a contour of the cell aggregation.

19. The image processing device according to claim 16, **characterized in that** the characteristic relating to stratification is a statistic based on contour shape of the cell aggregation.

20. A method for producing a cell aggregation, comprising:
a cell culture step of culturing cells ; and
a classification step of viewing, using the image processing device according to any of claims 12 through 19, the cells cultured in the cell culture step, and classifying cell aggregations of the cells changed by cell culturing.

21. A method for producing a cell aggregation, comprising:
a cell culture step of culturing cells;
a first extraction step of photographing, through use of an imaging device, the cells cultured in the cell culture step and extracting cells included in the image from a first image taken at a predetermined time point;
a second extraction step of extracting cells included in the image from a second image taken a predetermined time apart from the predetermined time point;
an information assigning step of associating the cells extracted from the first image and the cells extracted from the second image, assigning pre-integration cell information to the integrated cell in the case that a plurality of cells of the first image is integrated into a single cell in the second image, and assigning pre-separation cell information to the separated cells in the case that a single cell of the first image is separated into a plurality of cells in the second image;
an inheritance step of photographing the cells cultured in the cell culture step at predetermined time intervals through use of the imaging device and obtaining time-lapse images, executing the extraction and association of cells while sequentially allocating the time-lapse images as the first and second images along the time axis, and causing the cell information of the cells included in the images to be sequentially inherited; and
a classification step of classifying cell aggregations on the basis of the inherited cell information for the cells included in an image taken at an arbitrary time point.
